# EUROPÄISCHE PATENTSCHRIFT

(11) **EP 2 144 917 B1**
(45) Veröffentlichungstag und Bekanntmachung des Hinweises auf die Patenterteilung: **05.09.2012**
(21) Anmeldenummer: 07847402.0
(22) Anmeldetag: 27.11.2007
(51) Int. Cl.: C07F 7/04, C11D 3/50, A61L 9/00

(54) **1-AZA-3,7-DIOXABICYCLO[3.3.0]OCTAN-VERBINDUNGEN UND MONOCYCLISCHE OXAZOLIDINE ALS PRO-FRAGRANCES**
1-AZA-3,7-DIOXABICYCLO[3.3.0]OCTANE COMPOUNDS AND MONOCYCLIC OXAZOLIDINES FROM PRO-FRAGRANCES
COMPOSÉS DE TYPE 1-AZA-3,7-DIOXABICYCLO[3.3.0]OCTANE ET OXAZOLIDINE MONOCYCLIQUE EN TANT QUE PRO-FRAGRANCES

(30) Priorität: 20.12.2006 DE 102006060943
(43) Veröffentlichungstag der Anmeldung: 20.01.2010
(73) Patentinhaber: Henkel AG & Co. KGaA, 40589 Düsseldorf (DE)
(72) Erfinder: HUCHEL, Ursula, 50670 Köln (DE); GERKE, Thomas, 40627 Düsseldorf (DE); SAUF, Silvia, 40235 Düsseldorf (DE); KLINK, Claudia, 47877 Willich 4 (DE)
(86) Internationale Anmeldenummer: PCT/EP2007/062875
(87) Internationale Veröffentlichungsnummer: WO 2008/074598

(56) Entgegenhaltungen:
- EP-A- 0 414 962
- US-A1- 2003 207 786

## Beschreibung

Die Erfindung betrifft Kieselsäureester, an denen Duftstoffe, vorzugsweise als 1-Aza-3,7-dioxabicyclo[3.3.0]octan-Verbindungen oder als monocyclische Oxazolidine, gebunden sind, und sich beispielsweise zur Beduftung von Wasch- und Reinigungsmitteln eignen, da sie bei der Hydrolyse die gebundenen Duftstoffe wieder freisetzen.

Die kontrollierte Freisetzung von Inhaltsstoffen in den unterschiedlichsten Präparaten, gerne als "controlled release" bezeichnet, ist Gegenstand zahlreicher Veröffentlichungen und Patentanmeldungen. Auf dem Gebiet der Wasch- und Reinigungsmittel sind dabei beschleunigte oder verzögerte Freisetzungen von Inhaltsstoffen aus der Gruppe der Bleichmittel, Bleichaktivatoren, Tenside usw. von besonderem Interesse. Von herausragender Bedeutung ist auf diesem Gebiet die Freisetzung von Duftstoffen, da sowohl das Produkt, als auch die Wasch- und Reinigungslösung und die mit diesen Mitteln behandelten Gegenstände intensiv und langanhaltend beduftet werden sollen.
Ähnliches gilt auch für kosmetische Mittel, wobei hier nicht die Beduftung eines Produktes, sondern die Beduftung von Haut oder Haar bewirkt werden soll.

Ein grundsätzliches Problem bei der Verwendung von Duftstoffen besteht hierbei darin, dass es sich bei diesen naturgemäß um flüchtige Substanzen handelt, ansonsten könnte kein Dufteffekt erzielt werden. Man steht daher bei dem Einsatz von Duftstoffen in Wasch- und Reinigungsmitteln ebenso wie bei dem Einsatz in kosmetischen Zubereitungen vor dem Problem, dass es sich bei den Duftstoffen naturgemäß um flüchtige Verbindungen handelt, man aber andererseits einen langanhaltenden und möglichst gleich bleibenden Dufteffekt bewirken möchte. Hinzu kommt des weiteren, dass sich der Dufteindruck eines Parfüms im Laufe der Zeit verändert, weil die Riechstoffe, die die frischen und leichten Noten des Parfüms darstellen durch ihren hohen Dampfdruck schneller abdampfen als die Duftstoffe, die die Herz- und Basisnoten darstellen.

Neben den Methoden, Duftstoffe auf Trägermaterialien aufzubringen und die bedufteten Träger zu beschichten, oder Duftstoffe zu verkapseln oder in Verbindungen einzulagern (beispielsweise Cyclodextrin-Parfüm-Komplexe), existiert die Möglichkeit, die Duftstoffe chemisch an Trägermedien zu binden, wobei die chemische Bindung langsam gespalten und der Duftstoff freigesetzt wird. Eine solche Träger-gebundene Vorform eines Duftstoffes wird auch als "Profragrance", "Pro-accord" oder "Duftspeicherstoft" bezeichnet.
Als Beispiele für die Überführung eines Duftstoffs in eine Träger-gebundene Vorform sei die Veresterung von Duftstoffalkoholen genannt, wobei zu dieser Stoffgruppe ein breiter Stand der Technik existiert.

Im Stand der Technik existieren einige Vorschläge, duftende Alkohole an nicht flüchtige Siloxane zu binden, aus denen sie durch Hydrolyse langsam freigesetzt werden. Obwohl auch zu Siloxanestern von Duftstoffalkoholen ein breiter Stand der Technik existiert, treten beim Einsatz der genannten Verbindungen in Wasch- und Reinigungsmitteln oftmals Probleme auf. So sind viele der bekannten Verbindungen in wässrigen Wasch- und Reinigungsmitteln nicht immer einsetzbar, da sie bereits im Produkt hydrolysieren und die verzögerte Freisetzung hierdurch bedingt später nicht mehr auftritt. Dies ist umso mehr der Fall, als übliche Wasch- und Reinigungsmittel oft pH-Werte aufweisen, die die Hydrolyse noch verstärken. Auch die Einarbeitung der bekannten Siloxanester in pulverförmigen Wasch- und Reinigungsmitteln ist nicht immer so einfach. Unter üblichen Herstellbedingungen verdichteter Teilchengemische wie Granulierung oder Pressagglomeration neigen die Siloxanester ebenfalls dazu, den Duftstoffalkohol bereits bei der Herstellung, d.h. verfrüht, freizusetzen.
Es besteht daher ein Bedürfnis, Duftstoff-Vorformen bereitzustellen, die sowohl das Produkt, beispielsweise Wasch- und Reinigungsmittel, als auch die mit den Produkten behandelten Substraten, insbesondere Textilien, möglichst langanhaltend beduften.

Monomere Orthokieselsäureester von Duftstoffalkoholen werden beispielsweise in der US 3,215,719 (Dan River Mills) beschrieben. Diese Schrift nennt auch die verzögerte Freisetzung duftender Alkohole aus gemischten Estern wie beispielsweise Bis(eugenyloxy)diethoxysilan oder Bis(cinnamoyloxy)diethoxysilan, wobei das zentrale Si nicht zwingend nur an Sauerstoff gebunden sein muß. Oligomere Siloxanester werden in dieser Schrift nicht beschrieben.

Pulverförmige oder granulatartige Wasch- und Reinigungsmittel-zusammensetzungen, die "Wohlgeruch verleihende" Silicium-Verbindungen enthalten, werden in der DE 28 44 789 (Dow Corning) -beschrieben. Die hierin offenbarten mono-, oligo- und polymeren Siliciumverbindungen weisen nicht zwingend ein von vier Sauerstoffatomen umgebenes Si-Zentralatom auf. Oligomere Si-Verbindungen mit mehr als einer Duftstoffalkohol-Estergruppe werden in dieser Schrift ebenfalls nicht beschrieben.

In US20030207786 und US20040067870 sind ebenfalls Profragrances beschrieben, die eine Oxazolidinstruktur aufweisen.

In der US 6,861,402 sind Pro-Fragrances beschrieben, die einen Duftaldehyd oder ein Duftketon in Form eines Oxazolidins gebunden enthalten. Dabei wird beispielsweise N-Benzolethanolamin mit einem Duftstoff umgesetzt, so dass sich ein monocyclisches Oxazolidin ergibt.

Aufgabe der vorliegenden Erfindung war es daher, Duftstoff-Vorformen, sogenannte "Profragrances", insbesondere für Aldehyd-Duftstoffe und Keton-Duftstoffe, bereitzustellen. Insbesondere bestand die Aufgabe darin, hydrolysestabile Siloxanester von Duftstoffen bereitzustellen, die sich auch in wässrige Wasch- und Reinigungsmittel einarbeiten lassen, ohne bereits im Produkt übermäßigen Hydrolyseerscheinungen zu unterliegen. Auch die Einarbeitbarkeit der Verbindungen in granulare Wasch- und Reinigungsmittelzusammensetzungen, ohne dass es im Herstellprozess zu Zersetzungen kommt, ist eine Anforderung an die bereitzustellenden Verbindungen.
Weiterhin sollen die mit den erfindungsgemäßen Verbindungen behandelten Substraten, einen angenehmen und langanhaltenden Duft erhalten.

Überraschenderweise wurde nun gefunden, dass Duftstoffe, die als 1-Aza-3,7-dioxabicyclo[3.3.0]octan-Verbindungen (bicyclische Oxazolidin-Derivate) und / oder als monocyclische Oxazolidine an Kieselsäureestern gebunden sind, auf einfache Weise für den Einsatz in Wasch- und Reinigungsmitteln sowie kosmetischen Zubereitungen sehr gut geeignete Profragrances erhalten werden.
Weiterhin wurde erfindungsgemäß gefunden, dass insbesondere bicyclische und / oder monocyclische Oxazolidin-Derivate von Duftaldehyden und Duftketonen eine Verminderung des Dampfdrucks der Duftaldehyde und Duftketone und eine Verlängerung des Dufteindrucks erlauben. Zudem kann die Ablagerung der genannten Verbindungen dadurch auf festen Oberflächen wie Textilien, Haut oder harten Oberflächen verbessert werden.
Hierbei wurde herausgefunden, dass die Kieselsäureester vorzugsweise als Mischung von Estern anderer Oligokieselsäuren vorliegen. Unter den überraschenden Eigenschaften ist hervorzuheben, dass diese Verbindungen in Wasch- und Reinigungsmitteln sowie in Kosmetika weitgehend hydrolysestabil sind, dass jedoch bei Anwendung der erfindungsgemäßen Profragrances enthaltenden Wasch- und Reinigungsmittel sowie Kosmetika (kosmetische Mittel) in gewünschter Weise ein verzögerter release-Effekt bewirkt werden kann. Insbesondere kann etwa mit einem erfindungsgemäß Profragrance enthaltenden Weichspüler ein langanhaltender Dufteffekt auf der behandelten Wäsche erzielt werden, was möglicherweise darauf zurückzuführen ist, dass sich die erfindungsgemäßen Profragrances an der Wäsche anhaften und erst nach und nach unter Einwirkung der Luftfeuchtigkeit durch Hydrolyse die Freisetzung der Duftstoffe (Duftstoffaldehyde bzw. -ketone) erfolgt.

Gegenstand der vorliegenden Erfindung sind daher Kieselsäureester an denen Duftstoffe bzw. Riechstoffe gebunden vorliegen, die vorzugsweise durch Hydrolyse über einen längeren Zeitraum wieder freigesetzt werden und so ein langanhaltendes Duftempfinden bewirken. Die Duft- bzw. Riechstoffe liegen dabei vorzugsweise als bicyclische und / oder monocyclische Oxazolidinderivate vor.

Gegenstand der vorliegenden Erfindung sind demgemäß Kieselsäureester der allgemeinen Formel (I) 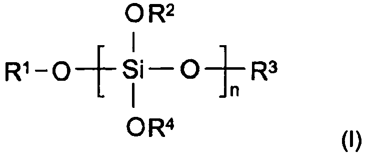 wobei mindestens einer der Reste R¹, R², R³ und R⁴ unabhängig voneinander
(a) eine 1-Aza-3,7-dioxabicyclo[3.3.0]octan-Verbindung der allgemeinen Formel (II), 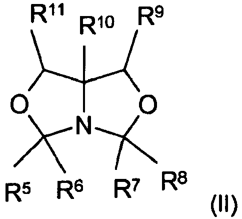
   mit R⁵, R⁶, R⁷, R⁸ unabhängig voneinander Wasserstoff, Alkylrest mit 1 bis 8 C-Atomen oder Reste, die in einer Verbindung der allgemeinen Formel
   R⁵-C(=O)-R⁶ bzw. R⁷-C(=O)-R⁸ einen Duftstoff, insbesondere einen Duftstoffaldehyd oder ein Duftstoffketon ergeben, wobei R⁵ und R⁶ bzw. R⁷ und R⁸ nicht gleichzeitig Wasserstoff sein können,
   R⁹, R¹¹ unabhängig voneinander Wasserstoff, Alkylrest mit 1 bis 8 C-Atomen, Hydroxyalkyl mit 1 bis 8 C-Atomen, Aminoalkyl,
   R¹⁰ eine Bindung oder ein divalenter Alkylenrest mit 1 bis 8 C-Atomen, wobei die Bindung der 1-Aza-3,7-dioxabicyclo [3.3.0] octan-Verbindung der allgemeinen formel II an die Kieselsäure ester über den Rest R¹⁰ erfolgt
   und / oder
(b) ein monocyclisches Oxazolidin der allgemeinen Formel (III) darstellt, 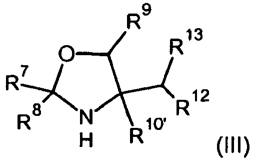
   mit den vorstehend angegebenen Bedeutungen für die Reste R⁷, R⁸, R⁹ und
   R^{10'} eine Bindung oder ein divalenter Alkylenrest mit 1 bis 8 C-Atomen, Wasserstoff, Alkylrest, das durch ein oder zwei Hydroxylgruppen und/oder eine Aminogruppe substituiert sein kann und/oder in dem bis zu 8 nicht benachbarte -CH₂-Gruppen durch - O- ersetzt sein können,
   und R¹² Wasserstoff, Alkylrest mit 1 bis 8 C-Atomen, Hydroxyalkyl mit 1 bis 8 C-Atomen oder Aminoalkyl,
   R¹³ eine OH-Gruppe oder eine Bindung,
und die noch verbleibenden Reste R¹, R², R³ und R⁴ unabhängig voneinander ausgewählt aus Wasserstoff, Alkylrest mit 1 bis 20 C-Atomen oder Riechstoffalkoholrest,
und n Werte aus dem Bereich von 2 bis 20 annimmt, wobei die Bindung des monocyclischen Oxazolidins der allgemeinen Formel (III) an die Kieselsaüreester über den Rest R¹⁰¹ und oder über den Rest R¹³ erfolgt.

In den Verbindungen der allgemeinen Formel (I) in denen die Reste R¹, R², R³ und / oder R⁴ unabhängig voneinander Verbindungen der Formeln (II) und / oder (III) bedeuten können, bedeuten R⁵, R⁶, R⁷ und R⁸ unabhängig voneinander Reste, die in einer Verbindung der allgemeinen Formel
R⁵-C(=O)-R⁶ bzw. R⁷-C(=O)-R⁸ vorzugsweise einen Duftstoffaldehyd oder ein Duftstoffketon ergeben.
Dabei können R⁵ und R⁶ bzw. R⁷ und R⁸ in Formel (II) nicht gleichzeitig Wasserstoff sein.

Vorzugsweise weisen in dem Strukturelement -CR⁵R⁶ die Reste R⁵ und R⁶ und in dem Strukturelement -CR⁷R⁸ die Reste R⁷ und R⁸ jeweils zusammen mindestens sechs C-Atome, vorzugsweise mindestens fünf C-Atome auf.
In den Verbindungen der allgemeinen Formel (I) liegen somit vorzugsweise Duftstoffaldehyde und/oder Duftstoffketone als 1-Aza-3,7-dioxabicyclo[3.3.0]octan-Verbindung (bicyclisches Oxazolidin) der allgemeinen Formel (II) und / oder als monocyclisches Oxazolidin der allgemeinen Formel (III) derivatisiert vor.
Als Duftstoffaldehyde oder Duftstoffketone können dabei alle üblichen Duftstoffaldehyde und Dufstofftketone eingesetzt werden, die typischerweise zur Herbeiführung eines angenehmen Duftempfindens eingesetzt werden.

Duftstoffe und Riechstoffe sind im Rahmen der vorliegenden Erfindung als Synonym zu verstehen.

Erfindungsgemäß sind "Duftstoffketone" Duftstoffe, die über mindestens eine freie Ketogruppe verfügen. Es können auch Gemische unterschiedlicher Ketone eingesetzt werden. Bevorzugt sind Duftstoffketone ausgewählt aus der Gruppe, umfassend Buccoxim, Iso jasmon, Methyl beta naphthyl keton, Moschus indanon, Tonalid/Moschus plus, Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro-Beta-Ionon, Fleuramon, Dihydrojasmon, Cis-Jasmon, Iso-E-Super, Methyl-cedrenylketon oder Methyl-cedrylon, Acetophenon, Methyl-acetophenon, Para-Methoxy-acetophenon, Methyl-beta-naphtyl-keton, Benzyl-aceton, Benzophenon, Para-Hydroxy-phenyl-butanon, Celery Keton oder Livescon, 6-Isopropyldecahydro-2-naphton, Dimethyl- octenon, Freskomenth, 4-(I-Ethoxyvinyl)-3,3,5,5,-tetramethyl-cyclohexanon, Methyl-heptenon, 2-(2-(4-Methyl-3-cyclohexen-1-yl)propyl)-cyclopentanon, 1-(p-Menthen-6(2)-yl-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl-cyclocitron, Methyl-Lavendel-keton, Orivon, Para-tert-butylcyclohexanon, Verdon, Delphon, Muscon, Neobutenon, Plicaton, Velouton, 2,4,4,7-Tetramethyl-oct-6-en-3-on, Tetrameran, Hedion und Gemischen davon. Bevorzugt können die Ketone ausgewählt sein aus Alpha Damascon, Delta Damascon, Iso Damascon, Carvon, Gamma-Methyl-ionon, Iso-E-Super, 2,4,4,7-Tetramethyl-oct-6-en-3-on, Benzylaceton, Beta Damascon, Damascenon, Methyldihydrojasmonat, Methyl-cedrylon, Hedion und Gemischen davon.

Erfindungsgemäß sind "Duftstoffaldehyde" Duftstoffe, die über mindestens eine freie Aldehydgruppe verfügen. Geeignete Duftstoffaldehyde können beliebige Aldehyde sein, die entsprechend der Duftstoffketone einen gewünschten Duft oder ein Frischeempfinden vermitteln. Es kann sich wiederum um einzelne Aldehyde oder Aldehydgemische handeln. Aus der großen Gruppe der Duftstoffaldehyde lassen sich folgende bevorzugte Vertreter nennen: Octanal, Citral, Melonal, Lilial, Floralozon, Canthoxal, 3-(4-Ethylphenyl)-2,2-Dimethylpropanal, 3-(4-Methoxyphenyl)-2-methylpropanal, 4-(4-Hydroxy-4-methylpentyl)-3-cyclohexen-1-carboxaldehyd, Phenylacetaldehyd, Methylnonylacetaldehyd, 2-Phenylpropan-1-al, 3-Phenylprop-2-en-1-al, 3-Phenyl-2-pentylprop-2-en-1-al, 3-Phenyl-2-hexylprop-2-enal, 3-(4-Isopropylphenyl)-2-methylpropan-1-al, 3-(4-Ethylphenyl)-2,2-dimethylpropan-1-al, 3-(4-tert-Butylphenyl)-2-methyl-propanal, 3-(3,4-Methylendioxyphenyl)-2-methylpropan-1-al, 3-(4-Ethylphenyl)-2,2-dimethylpropanal, 3-(3-Isopropylphenyl)butan-1-al, 2,6-Dimethylhept-5-en-1-al, n-Decanal, n-Undecanal, n-Dodecanal, 3,7-Dimethyl-2,6-octadien-1-al, 4-Methoxybenzaldehyde, 3-Methoxy-4-hydroxybenzaldehyde, 3-Ethoxy-4-hydroxybenzaldehyde, 3,4-Methylendioxybenzaldehyd und 3,4-Dimethoxybenzaldehyd, Adoxal, Anisaldehyd, Cumal, Ethylvanillin, Florhydral, Helional, Heliotropin, Hydroxycitronellal, Koavon, Laurylaldehyd, Lyral, Methylnonylacetaldehyd, Bucinal, Phenylacetaldehyd, Undecylenaldehyd, Vanillin, 2,6,10-Trimethyl-9-undecenal, 3-Dodecen-1-al, alpha-n-Amylcinnamaldehyd, 4-Methoxybenzaldehyd, Benzaldehyd, 3-(4-tert.Butylphenyl)-propanal, 2-Methyl-3-paramethoxyphenylpropanal, 2-Methyl-4-(2,6,6-trimethyl-2(I)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-dimethyl-6-octenyl)oxy]acetaldehyd, 4-Isopropylbenzaldehyd, 1,2,3,4,5,6,7,8-Octahydro-8,8-dimethyl-2-naphthaldehyd, 2,4-Dimethyl-3-cyclohexen-1-carboxaldehyd, 1-Decanal, Decylaldehyd, 2,6-Dimethyl-5-heptenal, 4-(Tricyclo[5.2.1.0(2,6)]-decyliden-8)-butanal, Octahydro-4,7-methano-1-indencarboxaldehyd, 3-Ethoxy-4-hydroxybenzaldehyd, para-Ethyl-alpha,alpha-dimethylhydrocinnamaldehyd, alpha-Methyl-3,4-(methylendioxy)-hydrocinnamaldehyd, 3,4-Methylendioxybenzaldehyd, alpha-n-Hexylcinnamaldehyd, m-Cumen-7-carboxaldehyd, alpha-Methylphenylacetaldehyd, 7-Hydroxy-3,7-dimethyloctanal, Undecenal, 2,4,6-trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert.butyl)propanal, Dihydrocinnamaldehyd, 1-Methyl-4-(4-methyl-3-pentenyl)-3-cyclohexen-1-carboxaldehyd, 5- oder 6-Methoxyhexahydro-4,7-methanoindan-1 oder 2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxycinnamaldehyd, 3,5,6- Trimethyl-3-cyclohexencarboxaldehyd, 3,7-Dimethyl-2-methylen-6-octenal, Phenoxyacetaldehyd, 5,9-Dimethyl-4,8-decadienal, Peonyaldehyd (6,10-Dimethyl-3-oxa-5,9-undecadien-1-al), Hexahydro-4,7-methanoindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal und Methylnonylacetaldehyd.

Für weitere geeignete Duftstoffe, ausgewählt aus Aldehyden und Ketonen, wird auf ***Steffen Arctander*** Published 1960 and 1969 respectively, Reprinted 2000 ISBN: Aroma Chemicals Vol. 1: 0-931710-37-5, Aroma Chemicals Vol. 2: 0-931710-38-3, verwiesen.

Unter "Riechstofffalkoholreste" werden im Rahmen der vorliegenden Erfindung Riechstoffe verstanden, die über freie Hydroxylgruppen verfügen, welche veresterbar sind, unabhängig davon, wie das Molekül weiter aufgebaut ist. So lassen sich auch Salicylsäureester als Riechstofffalkohole einsetzen. Aus der großen Gruppe der Riechstofffalkohole lassen sich bevorzugte Vertreter nennen, so dass im Rahmen der vorliegenden Erfindung Kieselsäureester bevorzugt sind, in denen R¹, R², R³, R⁴ unabhängig voneinander ausgewählt sein kann aus der Gruppe der folgenden Riechstofffalkohole: 10-Undecen-1-ol, 2,6-Dimethylheptan-2-ol, 2-Methylbutanol, 2-Methylpentanol, 2-Phenoxyethanol, 2-Phenylpropanol, 2-tert.-Butycyclohexanol, 3,5,5-Trimethylcydohexanol, 3-Hexanol, 3-Methyl-5-phenylpentanol, 3-Octanol, 3-Phenylpropanol, 4-Heptenol, 4-Isopropylcyclo-hexanol, 4-tert.-Butycyclohexanol, 6,8-Dimethyl-2-nonanol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol.

Unter "Alkylreste" werden im Rahmen der vorliegenden Erfindung Alkyle verstanden, die substituiert oder unsubstituiert, verzweigt oder linear sind. Bevorzugt sind efindungsgemäß Alkyle mit 1 bis 20 C-Atomen, besonders bevorzugt 1 bis 8 C-Atomen und ganz besonders bevorzugt 1 bis 4 C-Atomen. Bevorzugte Alkylreste sind beispielsweise Methyl, Ethyl, Propyl, iso-Propyl, Butyl, tert-Butyl, Pentyl, Hexyl, Heptyl, Octyl, wobei diese noch weiter substituiert oder unsubstituiert sein können. Insbesondere bevorzugt sind Methyl, Ethyl, Propyl, iso-Propyl, Octyl, die substituiert oder unsubstituiert sein können.

Unter "divalente Alkylenreste" werden im Rahmen der vorliegenden Erfindung Alkylreste verstanden, die an beiden Seiten noch eine Bindung eingehen können, so dass beispielsweise auf der einen Seite die Bindung zum bicyclischen Oxazolidin (1-Aza-3,7-dioxabicyclo[3.3.0]octan-Verbindung der allgemeinen Formel (II)) und auf der anderen Seite die Bindung zum Sauerstoffatom der Verbindung der allgemeinen Formel (I) erfolgt. Erfindungsgemäß weist ein solcher erfindungsgemäßer Kieselsäureester beispielsweise die folgende allgemeine Struktur auf: 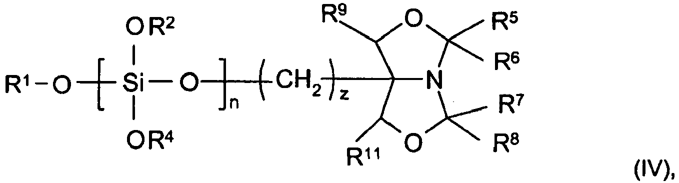 wobei die Reste R¹, R², R⁴, R⁵, R⁶, R⁷, R⁸, R⁹ und R¹¹ und n die vorstehenden angegebenen Bedeutungen besitzen und z eine Zahl von 0 bis 8 annimmt, bevorzugt 1 bis 4, ganz besonders bevorzugt 1 oder 2.
Wie in Formel (IV) dargestellt, erfolgt somit die Bindung der 1-Aza-3,7-dioxabicyclo[3.3.0]octan-Verbindung der allgemeinen Formel (II) vorzugsweise über den Rest R¹⁰ an die Kieselsäureester der allgemeinen Formel (I). Hierbei stellt R¹⁰ den divalenten Alkylenrest dar, der bevorzugte substituiert oder unsubstituiert, gesättigt oder ungesättigt sein kann.
Bevorzugt sind divalente Alkylenreste mit 1 bis 8 C-Atomen. Bevorzugte Alkylenreste sind ausgewählt aus beispielsweise
-CH₂- (Methylen), -CH₂-CHr (Ethylen), -CH₂-CH₂-CH₂- (Propylen), -CH₂-CH₂-CH₂-CH₂-(Butylen), -CH₂-CH₂-CH₂-CH₂-CH₂- (Pentylen), -CH₂-CH₂-CH₂-CH₂-CH₂-CH₂- (Hexylen), -CH₂-CH₂-CH₂-CH₂-CH₂-CH₂-CH₂- (Heptylen), -CH₂-CH₂-CH₂-CH₂-CH₂-CH₂-CH₂-CH₂-(Octylen), aber auch die verzweigten Derivate davon, wie iso-Propylen, tert-Butylen, besonders bevorzugt sind Methylen, Ethylen sowie Propylen bzw. iso-Propylen, ganz besonders bevorzugt Methylen und Ethylen.

Unter "Bindung" wird im Rahmen der vorliegenden Erfindung eine einfache Bindung verstanden, die beispielsweise eine Bindung zwischen dem Molekül gemäß der allgemeinen Formel (II) oder (111) mit der Verbindung gemäß der allgemeinen Formel (I) bildet. Erfindungsgemäß kann ein weiterer erfindungsgemäßer Kieselsäureester demgemäß beispielsweise die folgende allgemeine Struktur aufweisen: 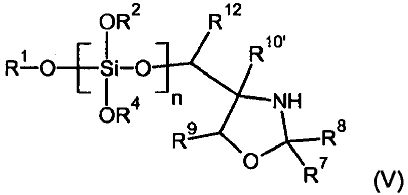 wobei die Reste R¹, R², R⁴, R⁷, R⁸, R⁹, R^{10'} und R¹² und n die vorstehenden angegebenen Bedeutungen besitzen.
Wie in Formel (V) dargestellt, erfolgt die Bindung der Verbindung (III) an (I) vorzugsweise über R¹³, welche in diesem Fall lediglich eine Bindung darstellt.
Ebenfalls ist es möglich, dass die Bindung über R^{10'} erfolgt, welches dann bevorzugt ein divalenter Alkylenrest ist. Ein solcher erfindungsgemäßer Kieselsäureester ist beispielsweise durch die allgemeine Struktur (VI) dargestellt: 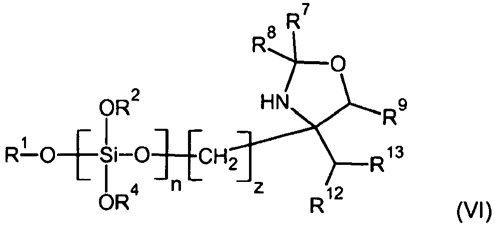 wobei die Reste R¹, R², R⁴, R⁷, R⁸, R⁹ und R¹² und n die vorstehenden angegebenen Bedeutungen besitzen und z eine Zahl von 0 bis 8 annimmt, bevorzugt 1 bis 4, ganz besonders bevorzugt 1 oder 2,
und R¹³ in diesem Fall vorzugsweise eine OH-Gruppe darstellt.

Unter "Hydroxyalkyle" werden im Rahmen der vorliegenden Erfindung Alkylreste verstanden, die an einer beliebigen Stelle mit mindestens einer Hydroxy-Gruppe (-OH) substituiert ist. Vorzugsweise weist ein solches Hydroxyalkyl mindestens ein, bevorzugt zwei OH-Gruppen auf, wobei die bevorzugten Alkylreste bereits oben beschrieben wurden.

Unter "Aminoalkyle" werden im Rahmen der vorliegenden Erfindung Alkylreste verstanden, die an einer beliebigen Stelle mit mindestens einer Amino-Gruppe (-NH₂) substituiert ist. Die Aminogruppe kann wiederum substituiert sein, so dass nicht zwangsläufig ein endständiges (primäres) Amin vorliegt. Vorzugsweise weist ein solches Aminoalkyl mindestens ein, bevorzugt zwei Amino-Gruppen auf.

Die erfindungsgemäßen Kieselsäureester werden vorzugsweise als Profragrances eingesetzt. Wie bereits oben erklärt, sind Profragrances träger-gebundene Vorformen eines Duftstoffes, wobei die chemische Bindung des derivatisierten Duftstoffes langsam gespalten und der Duftstoff wieder freigesetzt wird.
In einer bevorzugten Ausführungsform zerfällt daher mindestens eines der Strukturelemente -CR⁵R⁶ oder-CR⁷R⁸ der allgemeinen Formel (II) in einem solchen erfindungsgemäßen Kieselsäureester in eine Verbindung der allgemeinen Formel
R⁵-C(=O)-R⁶ oder R⁷-C(=O)-R⁸, welche die Eigenschaft eines Duftstoffes aufweist. Bevorzugt zerfallen beide Strukturelemente -CR⁵R⁶ und -CR⁷R⁸ in Formel (II) in Verbindungen der allgemeinen Formel R⁵-C(=O)-R⁶ und R⁷-C(=O)-R⁸, welche Duftstoffeigenschaften aufweisen. In einer weiteren bevorzugten Ausführungsform bedeuten R⁵, R⁷, R⁹, R¹¹ Wasserstoff und R⁵ und R⁶ in dem Strukturelement -CR⁵R⁶ und R⁷ und R⁸ in dem Strukturelement -CR⁷R⁸ jeweils zusammen mindestens sechs C-Atome, vorzugsweise mindestens fünf C-Atome auf.

In einer weiteren bevorzugten Ausführungsform zerfällt das Strukturelement -CR⁷R⁸ der allgemeinen Formel (III) in einem erfindungsgemäßen Kieselsäureester, welche mindestens ein monocyclisches Oxazolidin der allgemeinen Formel (III) aufweist, in eine Verbindung der allgemeinen Formel R⁷-C(=O)-R⁸, welche die Eigenschaft eines Duftstoffes aufweist.

In einer weiteren bevorzugten Ausführungsform der vorliegenden Erfindung liegen ein Teil der Riechstoffe, derivatisiert als 1-Aza-3,7-dioxabicyclo[3.3.0]octan-Verbindung der allgemeinen Formel (II) und / oder als monocyclisches Oxazolidin der allgemeinen Formel (III), welche an die Kieselsäureester der allgemeinen Formel (I) gebunden sind, vor. Vorzugsweise sind es Duftstoffaldehyde und -ketone, die in einer solche erfindungsgemäße trägergebundene Vorform eingesetzt werden.
Möglich ist jedoch auch, dass ein Teil der Riechstoffe, bevorzugt Riechstoffalkhohole, direkt an die Kieselsäureester verestert (beispielsweise R¹ = Geranyl) werden.
Diese trägergebundenen Vorformen eines Duftstoffes können selbstverständlich auch in einem Molekül (Formel (I)) gleichzeitig vorliegen.
Erfindungsgemäß können so unterschiedliche Duftstoffe, für die eine verzögerte Duftstofffreisetzung erwünscht wird (Aldehyde, Ketone, Alkohole), direkt und / oder als 1-Aza-3,7-dioxabicyclo[3.3.0]octan-Verbindung (beispielsweise Struktur IV) an Kieselsäureester der allgemeinen Formel (I) gebunden werden.
Ebenfalls können gleichzeitig Duftstoffaldehyde und -ketone auch als Verbindung der allgemeinen Formel (III) an die Kieselsäureester gebunden vorliegen.

In einer weiteren bevorzugten Ausführungsform besteht ein erfindungsgemäßes Profragrance aus der Kombination von Struktur (V) mit Struktur (V1). Vorzugsweise ist in einem solchen Fall beispielsweise der Rest R^{10'} (in Struktur (V)) wiederum ein divalenter Alkylenrest an dem eine Kieselsäureester gebunden vorliegt (analog Struktur (VI)), welche wiederum weitere Duftstoffe auf unterschiedlicher Weise enthalten kann, so dass eine quervernetzende Struktur entsteht.

Die erfindungsgemäßen Kieselsäureester können als alleiniger Duftstoff eingesetzt werden, es ist aber auch möglich, Duftstoffgemische einzusetzen, die lediglich zu einem Teil aus den erfindungsgemäßen Profragrances bestehen. Solche Gemische haben den Vorteil, dass auch die Bestandteile des Duftstoffgemischs, die nicht in Form von Kieselsäureester vorliegen, in der Haltbarkeit des Dufteindrucks verbessert werden. So können insbesondere Duftstoffgemische eingesetzt werden, die 1 bis 50 Gew.-%, vorzugsweise 5 bis 40 Gew.-% und insbesondere maximal 30 Gew.-% an Kieselsäureester enthalten. In anderen Ausführungsformen, bei denen insbesondere die verzögerte Duftwirkung der Trägergebundenen Form genutzt werden soll, werden bei der erfindungsgemäßen Verwendung vorteilhaft mindestens 30 Gew.-%, vorzugsweise mindestens 40 Gew.-% und insbesondere mindestens 50 Gew.-% des insgesamt in einem Mittel enthaltenen Parfüms über die erfindungsgemäßen Profragrances in das Mittel eingebracht, während die restlichen 70 Gew.-%, vorzugsweise 60 Gew.-% und insbesondere 50 Gew.-% des insgesamt im Mittel enthaltenen Parfüms auf übliche Weise aufgesprüht oder anders in das Mittel eingebracht werden. Die erfindungsgemäße Verwendung kann also vorteilhaft dadurch gekennzeichnet sein, dass die erfindungsgemäßen Kieselsäureester zusammen mit anderen Duftstoffen eingesetzt werden.

Durch die Aufteilung des Gesamt-Parfümgehaltes eines Mittels, beispielsweise eines Wasch-oder Reinigungsmittels, in Parfüm, welches in Form der erfindungsgemäßen Kieselsäureestern enthalten ist und Parfüm, das herkömmlich eingearbeitet wurde, lässt sich eine Vielzahl von Produktcharakteristiken realisieren, die erst durch die erfindungsgemäße Verwendung möglich werden. So ist es beispielsweise denkbar und möglich, den Gesamt-Parfümgehalt der Mittel in zwei Portionen x und y aufzuteilen, wobei der Anteil x aus erfindungsgemäßen Kieselsäureestern und der Anteil y aus herkömmlichen Parfümölen besteht.

Die einzige Grenze bei den erfindungsgemäßen Kieselsäureestern als Profragrances ist, dass die Duftstoffe, die über die erfindungsgemäßen Kieselsäureestern eingebracht werden, aus der Gruppe der Duftstoffaldehyde oder-ketone (wenn als 1-Aza-3,7-dioxabicyclo[3.3.0]octan-Verbindung oder als monocyclisches Oxazolidin an die Kieselsäureester gebunden) oder Riechstoffalkhole (wenn direkt an die Kieselsäureester gebunden) stammen müssen.

Die Duftstoffe, die auf herkömmliche Weise in die Mittel inkorporiert werden, sind dagegen keinerlei Beschränkungen unterworfen. So können als Parfümöle bzw. Duftstoffe einzelne Riechstoffverbindungen natürlichen oder synthetischen Ursprungs, z.B. vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 - 18 C-Atomen, Citral, Citronellal, Citronellyloxyacetaldehyd, Cyclamenaldehyd, Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.
Weitere herkömmliche Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bomylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanilin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Die Herstellung der erfindungsgemäßen Kieselsäureester gelingt durch einfache Umesterung von Oligokieselsäureestern niederer Alkohole mit Hydroxygruppen enthaltenden 1-Aza-3,7-dioxabicyclo[3.3.0]octan-Verbindungen bzw. monocyclischen Oxazolidinen.
Dabei bilden sich während der Veresterung in Nebenereaktionen vorzugsweise Kieselsäureringe. Bevorzugt bilden sich dabei insbesondere Ringe, die 3 bzw. 4 Siliciumatome enthalten. Dementsprechend liegt in einer weiteren bevorzugten Ausführungsform neben Kieselsäureester der allgemeinen Formel (I) eine Mischung aus Kieselsäureestern der Formel (VII) vor, 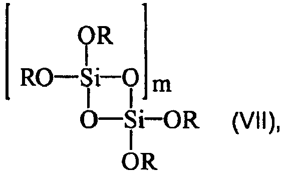 wobei die Reste R unabhängig voneinander die Bedeutungen der Reste R¹, R², R³ und R⁴ gemäß Formel (I) annehmen und m eine Zahl von 2 bis 20 ist.
Vorzugsweise enthält diese genannte Mischung Kieselsäureester der Formeln (VIII) und / oder (IX), 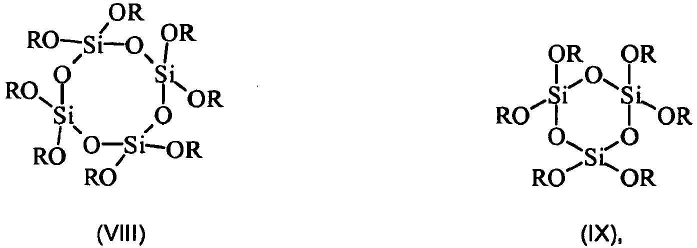 wobei die Reste R in den Formeln (VIII) und (IX) unabhängig voneinander die Bedeutungen der Reste R¹, R², R³ und R⁴ gemäß Formel (I) annehmen.

Kieselsäureester der allgemeinen Formel (I) umfassen daher vorzugsweise eine Mischung aus unterschiedlichen Kieselsäureestern, die sowohl eine cyclische Struktur (Formel VII, VIII, IX) als auch eine lineare Struktur (Formel I) oder eine Mischung aus beiden Strukturtypen, aufweisen können.
Letzteres ergibt sich beispielsweise, wenn mindestens einer der Reste
R in Formel (VII), (VIII) und (IX) wiederum ein Kieselsäureester der allgemeinen Formel (I) darstellt, wobei R dann den Platz von R¹, R², R³ und / oder R⁴ von Formel (I) annimmt. Vorzugsweise stellen mindestens die Hälfte alle Reste R in Formel (VII), (VIII) und (IX) Kieselsäureester der allgemeinen Formel (I) dar.

In einer bevorzugten Ausführungsform stellt mindestens einer der Reste, besonders bevorzugt mindestens zwei der Reste R¹, R², R³, R⁴ eine 1-Aza-3,7-dioxabicyclo[3.3.0]octan-Verbindung der allgemeinen Formel (II) dar.
Bevorzugt gilt dies auch für die Kieselsäureester der allgemeinen Formeln (VII), (VIII) und (IX), dass mindestens einer der Reste R die Bedeutung von R¹, R², R³, R⁴ annimmt und eine 1-Aza-3,7-dioxabicyclo[3.3.0]octan-Verbindung der allgemeinen Formel (II) darstellt. Ganz besonders bevorzugt stellen mindestens zwei, drei oder vier der Reste eine 1-Aza-3,7-dioxabicyclo[3.3.0]octan-Verbindung der allgemeinen Formel (II) dar.

Vorzugsweise weisen die erfindungsgemäßen Kieselsäureester dabei als Struktur dieselbe 1-Aza-3,7-dioxabicyclo[3.3.0]octan-Verbindungen der allgemeinen Formel (II) für die Reste R, R¹, R², R³, R⁴ auf.
Es ist jedoch auch möglich, dass die Reste R, R¹, R², R³ und R⁴ der erfindungsgemäßen Kieselsäureester voneinander unterschiedliche 1-Aza-3,7-dioxabicyclo[3.3.0]octan-Verbindungen der allgemeinen Formel (II) darstellen.

Dabei erfolgt die Bindung der 1-Aza-3,7-dioxabicyclo[3.3.0]octan-Verbindung der allgemeinen Formel (II) vorzugsweise durch den Rest R¹⁰ an die Kieselsäureestern der allgemeinen Formeln (I) (VII), (VIII) und (IX). Vorzugsweise stellt der Rest R¹⁰ dabei einen divalenten Alkylenrest dar, bevorzugt Methylen (-CH₂-) oder Ethen (-CH₂-CH₂-), besonders bevorzugt Methylen (siehe beispielsweise Formel IV, mit z = 1 oder 2).

Die Anzahl der derivatisierten Duftstoffe in einem Molekül bestimmt die Anzahl der Duftstoffe, die später frei gesetzt werden können. Dementsprechend ist es von Vorteil für ein lang anhaltendes Duftempfinden, wenn in einem Molekül möglichst viele Duftstoffe derivatisiert vorliegen, die nach und nach durch Hydrolyse freigesetzt werden können.
Daher ist es bevorzugt, dass mindestens eines der Strukturelemente -CR⁵R⁶ oder-CR⁷R⁸ in einem erfindungsgemäßen Kieselsäureester der allgemeinen Formel (I) mit Verbindungen der allgemeinen Formel (II) in eine Verbindung der allgemeinen Formel R⁵-C(=O)-R⁶ oder R⁷-C(=O)-R⁸ zerfällt, welche die Eigenschaft eines Duftstoffes aufweist. Bevorzugt zerfallen beide Strukturelemente -CR⁵R⁶ und -CR⁷R⁸ in Formel (II) in Verbindungen der allgemeinen Formel R⁵-C(=O)-R⁶ und R⁷-C(=O)-R⁸, welche Duftstoffeigenschaften aufweisen.

In einer weiteren bevorzugten Ausführungsform stellt mindestens einer der Reste, besonders bevorzugt mindestens zwei der Reste R¹, R², R³, R⁴ ein monocyclisches Oxazolidin der allgemeinen Formel (III) dar.
Bevorzugt gilt dies auch für die Kieselsäureester der allgemeinen Formeln (VII), (VIII) und (IX), dass mindestens einer der Reste R die Bedeutung von R¹, R², R³, R⁴ annimmt und ein monocyclisches Oxazolidin der allgemeinen Formel (III) darstellt. Ganz besonders bevorzugt stellen mindestens zwei, drei oder vier der Reste ein monocyclisches Oxazolidin der allgemeinen Formel (III) dar.

Vorzugsweise weisen die erfindungsgemäßen Kieselsäureester dabei als Struktur dieselbe monocyclische Oxazolidin-Struktur der allgemeinen Formel (III) für die Reste R, R¹, R², R³, R⁴ auf.
Es ist jedoch auch möglich, dass die Reste R, R¹, R², R³ und R⁴ der erfindungsgemäßen Kieselsäureester voneinander unterschiedliche monocyclische Oxazolidin-Strukturen der allgemeinen Formel (III) darstellen.

Vorzugsweise erfolgt dabei die Bindung des monocyclischen Oxazolidins der allgemeinen Formel (III) über den Rest R^{10'} an die Kieselsäureestern der allgemeinen Formeln (I) (VII), (VIII) und (IX). Vorzugsweise stellt der Rest R^{10'} dabei einen divalenten Alkylenrest dar, bevorzugt Methylen (-CH₂-) oder Ethen (-CH₂-CH₂-), besonders bevorzugt Methylen (siehe beispielsweise Formel VI, mit z = 1 oder 2).
Ebenfalls bevorzugt ist die Bindung des monocyclischen Oxazolidins der allgemeinen Formel (III) über den Rest R¹³ an die Kieselsäureestern der allgemeinen Formeln (I) (VII), (VIII) und (IX). Vorzugsweise stellt der Rest R¹³ dabei eine Bindung dar (siehe beispielsweise Formel V). Bevorzugt zerfällt das Strukturelement -CR⁷R⁸ in Formel (III) in solchen Fällen bei der Hydrolyse und somit der Freisetzung der Duftstoffe in Verbindungen der allgemeinen Formel R⁷-C(=O)-R⁸_{.}

Selbstverständlich ist es möglich, dass in einem erfindungsgemäßen Kieselsäureester beide Verbindungen der allgemeinen Formeln (II) und (III) enthalten sein kann. Ein solches erfindungsgemäßes Profragrance ist ebenfalls bevorzugt, da aus einem solchen Molekül mehrere Duftstoffe freigesetzt werden.

Die als 1-Aza-3,7-dioxabicyclo[3.3.0]octan-Verbindungen bzw. monocyclischen Oxazolidinen derivatisierten Duftstoffe sind vorzugsweise ausgewählt aus der Gruppe aus Jasmonen, lononen, Damasconen und Damascenonen, Menthon, Carvon, Iso-E-Super, Methyl-heptenon, Melonal, Cymal, Ethylvanilli, Helional, Hydroxycitronellal, Koavon, Methyl-nonylacetaldehyd, Phenylacetaldehyd, Undecylenaldehyd, 3-Dodecen-i-al, alpha-n-Amylzimtaldehyd, Benzaldehyd,
3-(4-tert-Butylphenyl)-propanal, 2-Methyl-3-(para-methoxyphenylpropanal), 2-Methyl-4-(2,6,6-trimethyl-2(1) -cyclohexen-1-yl)butanal, 3-Phenyl-2-propenal, cis-/trans-3,7-Dimethyl-2,6-octadien-1-al, 3,7-Dimethyl-6-octen-I-al, [(3,7-Dimethyl-6-octenyl)oxy]acetaldehyd, 4-Isopropylbenzyaldehyd, 2,4-Dimethyl-3-cyclohexen-1-carboxyaldehyd, 2-Methyl-3-(isopropylphenyl)propanal, Decylaldehyd, 2,6-Dimethyl-5-heptenal, alpha-n-Hexylzimtaldehyd, 7-Hydroxy-3,7-dimethyl octanal, Undecenal, 2,4,6-Trimethyl-3-cyclohexene-I-carboxaldehyd, 1-Dodecanal, 2,4-Dimethylcyclohexene-3-carboxaldehyd, 4-(4-Hydroxy-4-methyl pentyl)-3-cylohexene-I-carboxaldehyd, 2-Methyl undecanal, 2-Methyl decanal,
1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal,10-Undecen-1-al, 4-Hydroxy-3-methoxybenzaldehyd, trans-4-decenal, 2,6-Nonadienal, para-Tolylacetaldehyd, 3,7-Dimethyl-2-methylene-6-octenal, 2-Methyloctanal, alpha-Methyl-4-(I-methylethyl)benzeneacetaldehyd,
2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd, Citral, 1-Decanal, Florhydral, 2,4-Dimethyl-3-cyclohexen-1-carboxaldehyd, Heliotropin besonders bevorzugt.

Die Herstellung der erfindungsgemäßen Kieselsäureester gelingt, wie bereits erwähnt, durch einfache Umesterung von Oligokieselsäureestern niederer Alkohole mit Hydroxygruppen enthaltenden 1-Aza-3,7-dioxabicyclo[3.3.0]octan-Verbindungen bzw. monocyclischen Oxazolidinen, in dem vorzugsweise der Duftstoff bereits derivatisiert vorliegt. Reaktion A zeigt beispielsweise eine solche allgemeine Darstellung mit Hydroxygruppen enthaltenden 1-Aza-3,7-dioxabicyclo[3.3.0]octan-Verbindungen: 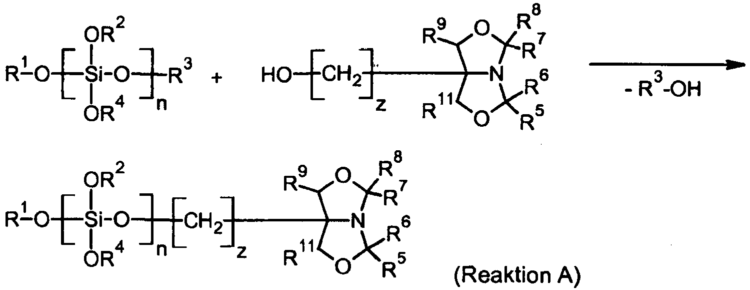 wobei die Reste R¹, R², R³, R⁴, R⁵, R⁶, R⁷, R⁸, R⁹ und R¹¹ und n und z die vorstehenden Bedeutungen besitzen.

Die Reaktion mit Hydroxygruppen enthaltenden monocyclischen Oxazolidinen erfolgt dementsprechend analog zu der Reaktion A.
Je nach Reaktionszeit und -bedingungen werden die niederen Alkohole abgespalten und die Hydroxygruppen enthaltenden 1-Aza-3,7-dioxabicyclo[3.3.0]octan-Verbindungen oder monocyclischen Oxazolidinen gebunden, wobei die Alkohole entlang von Si-O-Si-Ketten oder -Ringen leichter ausgetauscht werden als die terminalen Alkohole.
Derartige Umesterungen lassen sich beispielsweise, wie in der Publikation H. Steinmann, G. Tschernko, H. Hamann, Z. Chem. 3, 1977, S.89-92 beschrieben, durchführen.
Der Inhalt dieser Publikation wird ausdrücklich als Offenbarung dieser Anmeldung zur Herstellung von Kieselsäureestern angesehen. Üblicherweise werden als Edukte die handelsüblichen Kieselsäureester eingesetzt. Hier ist insbesondere der Ethanol-Ester zu nennen, der beispielsweise bei der Fa. Wacker, Burghausen erhalten werden kann. Die Umesterung kann dabei ausschließlich durch Temperaturerhöhung und Abdestillation der leichtflüchtigen Nebenprodukte gesteuert werden. Bevorzugt ist es jedoch, wenn zur Umesterung Katalysatoren eingesetzt werden. Es handelt sich dabei üblicherweise um Lewis-Säuren, vorzugsweise um Aluminiumtetraisopropylat, Titantetraisopropylat, Siliciumtetrachlorid oder basische Katalysatoren oder auch um Zubereitungen wie beispielsweise aus Aluminiumoxid mit Kaliumfluorid.
Die so gebildeten oligomeren Kieselsäureester weisen dann zumindest teilweise gebundene 1-Aza-3,7-dioxabicyclo[3.3.0]octan-Verbindungen der allgemeinen Formel (II) bzw. monocyclische Oxazolidine der allgemeinen Struktur (III) auf. Üblicherweise enthalten die resultierenden Ester jedoch auch noch Reste niederer Alkohole.
Soweit bei der Herstellung der Kieselsäureester geringe Mengen Wasser oder andere Wasserstoff-azide Verbindungen anwesend sind, findet auch Austausch von Alkohol-Resten gegen OH-Gruppen statt. Dementsprechend enthalten die erfindungsgemäßen Kieselsäureester üblicherweise als Rest (R¹, R², R³, R⁴ und/ oder R) teilweise auch Wasserstoff.
Oligokieselsäureester niederer Alkohole sind kommerziell erhältlich, wobei üblicherweise Methanol, Ethanol, n-Propanol, iso-Propanol, n-Butanol, iso-Butanol und tert.-Butanol zur Veresterung eingesetzt wurden.
Die Darstellung nicht vollständig mit 1-Aza-3,7-dioxabicyclo[3.3.0]octan-Verbindungen oder monocyclischen Oxazolidinen umgeesterter Oligokieselsäureester führt zu Kieselsäureestern in denen ein Teil der Reste R¹, R², R³, R⁴ und R (in Formel I, VII, VIII, IX) ausgewählt sind aus der Gruppe aus Methyl, Ethyl, n-Propyl, iso-Propyl, n-Butyl, iso-Butyl und tert.-Butyl. Ein weiterer Teil der Reste R¹, R², R³, R⁴ und R (in Formel I, VII, VIII, IX) des nicht vollständig umgeesterten Oligokieselsäureesters weist jedoch vorzugsweise die 1-Aza-3,7-dioxabicyclo[3.3.0]octan-Verbindungen gemäß der allgemeinen Formel (II) und / oder monocyclische Oxazolidine der allgemeinen Formel (III) auf, in denen die Strukturelemente - CR⁵R⁶ bzw. -CR⁷R⁸ in Verbindungen der allgemeinen Formel R⁵-C(=O)-R⁶ bzw.r R⁷_C(=0)-R⁸ hydrolysieren können, und so die Duftstoffe wieder freigesetzt werden.
Bevorzugt sind Verbindungen, in denen mindestens 50% der Reste R¹, R², R³, R⁴ und R der erfindungsgemäßen Kieselselsäureester bicyclische Oxazolidine (1-Aza-3,7-dioxabicyclo[3.3.0]octan-Verbindungen gemäß Formel (II)) oder monocyclische Oxazolidine (gemäß Formel (III)) sind.
Die vollständig umgeesterten Oligokieselsäureester sind im Rahmen der vorliegenden Erfindung besonders bevorzugt. Es sind Verbindungen, in denen jeder Rest R¹, R², R³, R⁴ und R (in Formel I, VII, VIII, IX) eine 1-Aza-3,7-dioxabicyclo[3.3.0]octan-Verbindung gemäß der allgemeinen Formel (II) und / oder ein monocyclisches Oxazolidin gemäß der allgemeinen Formel (III) darstellt.

Die Oligomerisierungsgrade "m" und "n" der erfindungsgemäßen Kieselsäureester liegen zwischen 2 und 20. In bevorzugten Verbindungen nimmt m bzw. n Werte zwischen 2 und 15, vorzugsweise zwischen 2 und 12 und insbesondere zwischen 3 und 10 unter besonderer Bevorzugung der Werte 4, 5, 6, 7 und 8, an.
Nimmt n beispielsweise in Formel (I) den Wert 2 an, so weist die resultierende Verbindung zwei -OR² und zwei -OR⁴ Einheiten auf. Erfindungsgemäß kann dann beispielsweise ein R² Rest (im ersten -OR²) eine 1-Aza-3,7-dioxabicyclo[3.3.0]octan-Verbindung oder ein monocyclisches Oxazoldin darstellen und der andere R² Rest (im zweiten -OR²) ein Wasserstoff, Alkylrest mit 1 bis 20 C-Atomen oder ein Riechstoffalkoholrest sein. Der R² Rest im zweiten -OR² kann jedoch auch wieder eine 1-Aza-3,7-dioxabicyclo[3.3.0]octan-Verbindung oder ein monocyclisches Oxazoldin sein.
Die beiden R⁴ Reste (in -OR⁴) sind ebenfalls unabhängig voneinander und können vorzugsweise unterschiedlich oder gleich sein.

Da die Ausgangsverbindungen für die Herstellung der erfindungsgemäßen Verbindungen aus ökonomischen Gründen vorzugsweise keine Reinstoffe sind, sondern technische Gemische von Oligokieselsäureestern niederer Alkohole mit unterschiedlichen Oligomerisierungsgraden, findet sich eine Verteilung der Oligomerisierungsgrade auch in den erfindungsgemäßen Estern wieder, die dem Ausgangsmaterial entsprechen kann oder durch die Reaktionsbedingungen modifiziert ist.

Die Herstellung der Verbindungen der allgemeinen Formel (II) erfolgt beispielsweise durch Umsetzung von Aminoalkoholen, vorzugsweise 2-Amino-1,3-propandiolderivate mit Aldehyden, Ketonen oder Mischungen von Ketonen und Aldehyden, die vorzugsweise durch die allgemeine Formel R⁵-C(=O)-R⁶ und R⁷-C(=O)-R⁸ beschrieben werden kann, unter Ringschluss. Die Umsetzung wird dabei vorzugsweise in einem geeigneten Lösungsmittel oder in situ durchgeführt. Geeignete Lösungsmittel sind beispielsweise aromatenhaltige Kohlenwasserstoffe wie Toluol. Die Umsetzung wird dabei vorzugsweise bei einer Temperatur im Bereich von 80 bis 150 °C, besonders bevorzugt 100 bis 140 °C durchgeführt. Beispielsweise wird der Aminoalkohol, unter Stickstoffatmosphäre zusammen mit dem gewünschten Keton oder Aldehyd im Lösungsmittel vorgelegt. Sodann wird das Reaktionsgemisch erhitzt, wobei der Feststoff langsam in Lösung geht. Häufig wird sodann unter Rückfluss am Wasserabscheider erhitzt. Das erhaltene Umsetzungsprodukt wird nach üblichen Verfahren isoliert und gegebenenfalls gereinigt.
Hierbei zeigte sich, dass bei der Umsetzung von geringeren als stöchiometrischen Mengen an Aldehyden und/oder Ketonen auch monocyclische Verbindungen im Produktgemisch vorliegen. Der Anteil an bicyclischen (Oxazolidin-) Verbindungen zu monocyclischen (Oxazolidin-) Verbindungen kann in einfacher Weise durch Auswahl des Molverhältnisses zwischen Aldehyd/Keton und 2-Amino-1,3-propandiol eingestellt werden.
Besonders bevorzugt sind Mischungen, die entweder einen hohen Anteil an bicyclischen oder monocyclischen Oxazolidinen aufweisen.
Vorzugsweise enthalten derartige Mischungen mindestens 50 Gew.%, vorzugsweise mindestens 65 Gew.-%, insbesondere mindestens 80 Gew.-% an bicyclischen Strukturen oder monocyclischen Oxazolidinen, je nach dem wie das Molverhältnis zwischen Aldehyd/Keton und 2-Amino-1,3-propandiol eingestellt wurde.

Liegt eine Mischung beispielsweise mit einen hohen Anteil an bicyclischen Oxazolidinen vor, so weist das erhaltene Umsetzungsprodukt, vor der Umesterung, in Position R¹⁰ (Formel (II)) vorzugsweise als Rest Wasserstoff oder einen Alkylrest auf, der durch ein oder zwei Hydroxylgruppen und/oder eine Aminogruppe substituiert sein kann und/oder in dem bis zu 8 nicht benachbarte -CH₂-Gruppen durch -O- ersetzt sein können. Dieses Umsetzungsprodukt wird dann anschließend mit Oligokieselsäureestern niederer Alkohole, wie bereits oben beschrieben, in die erfindungsgemäßen Kieselsäureester umgeestert.
Hierbei wird bevorzugt die Mischung eingesetzt, ohne dass die monocyclischen (Oxazolidin-) Verbindungen im Produktgemisch von den bicyclischen (Oxazolidin-) Verbindungen abgetrennt werden.

Die erfindungsgemäßen Kieselsäureester zeichnen sich durch eine gute Hydrolysestabilität aus und können auch in wässrigen Medien bzw. in Herstellprozessen für Granulate eingesetzt werden, ohne dabei übermäßige Aktivitätsverluste zu erleiden. Die erfindungsgemäßen Kieselsäureester werden dabei vorzugsweise als Profragrances, insbesondere zur Verlängerung der Duftwirkung von Duftstoffen eingesetzt.
Auf diese Weise sind flüssige Wasch- und Reinigungsmittel wie Flüssigwaschmittel, Weichspülmittel, Handgeschirrspülmittel, Reinigungsmittel für harte Oberflächen, Bodenwischmittel usw. ebenso denkbar wie feste Wasch- und Reinigungsmittel, beispielsweise Textilwaschmittelgranulate, maschinelle Geschirrspülmittel oder Putz- und Scheuermittel. Ebenso können die erfindungsgemäßen Kieselsäureester in kosmetischen Mitteln zur Haut- und Haarbehandlung eingesetzt werden. Auch hier sind dabei sowohl flüssige Mittel, wie beispielsweise Duschbäder, Deodorants und Haarshampoos, wie auch feste Mittel, wie beispielsweise Seifenstücke, gemeint.

Die erfindungsgemäßen Verbindungen der allgemeinen Formel (I), (VII), (VIII), (IX) werden erfindungsgemäß als Pro-Fragrances eingesetzt. Der Begriff "Pro-Fragrance" beschreibt dabei Derivate von Duftstoffaldehyden und Duftstoffketonen oder Riechstoffalkohole, die unter Umgebungsbedingungen die ursprünglichen Duftstoffaldehyde und Duftstoffketone oder Riechstoffalkohole freisetzen. Umgebungsbedingungen sind dabei die typischen Umgebungsbedingungen im menschlichen Lebensraum bzw. die auf der menschlichen Haut anzutreffenden Bedingungen. Unter Umgebungsbedingungen zerfallen die Verbindungen der allgemeinen Formel (I), (VII), (VIII), (IX) in Umkehr des Herstellungsverfahrens langsam unter Freisetzung der ursprünglichen Duftstoffaldehyde und Duftstoffketone bzw. Riechstoffalkohole. Die chemisch gebundenen Duftstoffaldehyde und Duftstoffketone oder Riechstoffalkohole werden an der Bindungsstelle gespalten, wodurch die Riechstoffe wieder freigesetzt werden.

Ein weiterer Gegenstand der vorliegenden Erfindung sind daher Wasch- oder Reinigungsmittel, enthaltend die erfindungsgemäßen Kieselsäureester.

In einer besonders bevorzugten Ausführungsform ist das Wasch- und Reinigungsmittel hierbei ein Weichspüler.

Die erfindungsgemäßen Kieselsäureester können dabei je nach Art und Verwendungszweck der zu beduftenden Mittel in variierenden Mengen eingebracht werden. Üblicherweise enthalten Wasch- oder Reinigungsmittel die erfindungsgemäßen Profragrances in Mengen von 0,001 bis 10 Gew.%, vorzugsweise von 0,01 bis 5 Gew.%, besonders bevorzugt von 0,02 bis 3 Gew.%, und insbesondere in Mengen von 0,05 bis 2 Gew.%, jeweils bezogen auf die Gesamtzusammensetzung des betroffenen Mittels.
Die Gesamtmenge der Duftstoffe in den erfindungsgemäßen Wasch- oder Reinigungsmitteln beträgt dagegen vorzugsweise zwischen 0,01 und 5 Gew.%, besonders vorzugsweise zwischen 0,1 und 3 Gew.-% sowie ganz besonders bevorzugt zwischen 0,5 und 2 Gew.-% bezogen auf die Gesamtmenge des Mittels. Bevorzugt werden Mischungen verschiedener Duftstoffe (aus den verschiedenen oben genannten Duftstoffklassen) verwendet, die gemeinsam eine ansprechende Duftnote erzeugen.
In diesem Fall ist die Gesamtmenge des mindestens einen Duftstoffs die Menge aller Duftstoffe in der Mischung zusammen bezogen auf die Gesamtmenge des Mittels.

Bevorzugt enthalten Wasch- oder Reinigungsmittel die erfindungsgemäßen Kieselsäureester in Mengen von 0,001 bis 10 Gew.-%, vorzugsweise von 0,01 bis 5 Gew.-%, besonders bevorzugt von 0,02 bis Gew.-% 3 und insbesondere in Mengen von 0,05 bis 2 Gew.-%, jeweils bezogen auf die Gesamtzusammensetzung des Wasch- und Reinigungsmittels.

In einer weiteren bevorzugten Ausführungsform handelt es sich bei den erfindungsgemäßen Mittel um pulverförmige oder granulatförmige Mittel.
Dabei können die teilchenförmigen Komponenten durch Sprühtrocknung, einfaches Mischen oder komplexe Granulationsverfahren, beispielsweise Wirbelschichtgranulation, hergestellt werden.

In einer weiteren bevorzugten Ausführungsform liegen die Wasch- oder Reinigungsmittel in Form von Formkörper vor, die nach einem Preßagglomerationsverfahren hergestellt werden, die Tablettierung, die sich in vier Abschnitten gliedert: Dosierung, Verdichtung (elastische Verformung), plastische Verformung und Ausstoßen.
Dementsprechend liegen die Wasch- oder Reinigungsmittel bevorzugt in Form von Formkörpern vor, wobei es sich vorzugsweise um Tabletten handelt, die aus einer einzigen oder aber aus mehren, insbesondere zwei oder drei verschiedenen Phasen bestehen können. Die Herstellung erfolgt dabei zunächst durch das trockene Vermischen der Bestandteile, die ganz oder teilweise vorgranuliert sein können, und anschließendes Informbringen, insbesondere Verpressen zu Tabletten, wobei auf herkömmliche Verfahren zurückgegriffen werden kann. Hierbei wird das Vorgemisch in einer sogenannten Matrize zwischen zwei Stempeln zu einem festen Komprimat verdichtet.

Um den Zerfall hochverdichteter Formkörper zu erleichtern, ist es möglich, Desintegrationshilfsmittel, sogenannte Tablettensprengmittel, in diese einzuarbeiten, um die Zerfallszeiten zu verkürzen.

Die Wasch- und Reinigungsmitteln können selbstverständlich weitere übliche Inhaltsstoffe von Wasch- und Reinigungsmittel enthalten. Vorzugsweise sind die üblichen Inhaltsstoffe ausgewählt aus der Gruppe der Tenside, Duftstoffen, Buildersubstanzen sowie Bleichmittel, Enzyme und andere Aktivstoffe zu nennen. Zu den wesentlichen Inhaltsstoffen von Waschund Reinigungsmitteln gehören dabei insbesondere Tenside.

Je nach Einsatzzweck der erfindungsgemäßen Mittel wird man den Tensidgehalt höher oder niedriger wählen. Üblicherweise liegt der Tensidgehalt von Waschmitteln zwischen 10 und 40 Gew.-%, vorzugsweise zwischen 12,5 und 30 Gew.-% und insbesondere zwischen 15 und 25 Gew.-%, während Reinigungsmittel für beispielsweise das maschinelle Geschirrspülen zwischen 0,1 und 10 Gew.%, vorzugsweise zwischen 0,5 und 7,5 Gew.-% und insbesondere zwischen 1 und 5 Gew.-% Tenside enthalten.

Diese grenzflächenaktiven Substanzen stammen aus der Gruppe der anionischen, nichtionischen, zwitterionischen oder kationischen Tenside, wobei anionische Tenside aus ökonomischen Gründen und aufgrund ihres Leistungsspektrums beim Waschen und Reinigen deutlich bevorzugt sind.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di-und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R¹⁴-O-(CH2-CH2O)x-CH2-COOH, in der R¹⁴ eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R¹⁵-O(CH₂-CH₂O)ₓ-OSO₃H, in der R¹⁵ eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 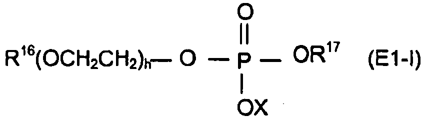 in der R¹⁶ bevorzugt für einen aliphatischen Kohlenwasserstoffrest mit 8 bis 30 Kohlenstoffatomen, R¹⁷ für Wasserstoff, einen Rest (CH₂CH₂O)ₙR¹⁸ oder X, h für Zahlen von 1 bis 10 und X für Wasserstoff, ein Alkali- oder Erdalkalimetall oder NR¹⁹R²⁰R²¹R²², mit R¹⁹ bis R²¹ unabhängig voneinander stehend für Wasserstoff oder einen C1 bis C4 - Kohlenwasserstoffrest, steht,
- sulfatierte Fettsäurealkylenglykolester der Formel (E1-II)

   R²²CO(AlkO)ₕSO₃M (E1-II)

   in der R²²CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, h für Zahlen von 0,5 bis 5 und M für ein Kation steht,
- Monoglyceridsulfate und Monoglyceridethersulfate der Formel (E1-III) 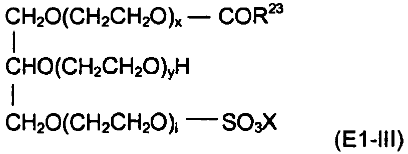 in der R²³CO für einen linearen oder verzweigten Acylrest mit 6 bis 22 Kohlenstoffatomen, x, y und i in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R²³CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobemsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Ebenfalls können kationische Tenside eingesetzt werden.
Erfindungsgemäß bevorzugt sind kationische Tenside vom Typ der quartären Ammonium-verbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quatemierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈- C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂- C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R²⁴CO-(OCH₂CHR²⁵)_{w}OR²⁶ (E4-I)

   in der R²⁴CO für einen linearen oder verzweigten, gesättigten und/oder ungesättigten Acylrest mit 6 bis 22 Kohlenstoffatomen, R²⁵ für Wasserstoff oder Methyl, R²⁶ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R²⁷O-[G]ₚ (E4-II)

   in der R²⁷ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl-und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R²⁷ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R²⁷ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 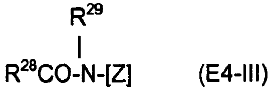 in der R²⁸CO für einen aliphatischen Acylrest mit 6 bis 22 Kohlenstoffatomen, R²⁹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R³⁰CO-NR³¹-CH₂-(CHOH)₄CH₂OH (E4-IV)

Vorzugsweise werden als Fettsäure-N-alkylpolyhydroxyalkylamide Glucamide der Formel (E4-IV) eingesetzt, in der R³¹ für Wasserstoff oder eine Alkylgruppe steht und R³⁰CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.
Diese Verbindungen sind durch die folgenden Parameter gekennzeichnet. Der Alkylrest enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "OxoAlkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Mittel können weiterhin mindestens einen Emulgator enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Eine andere bedeutende Gruppe von Wasch- und Reinigungsmittelinhaltsstoffen sind die Buildersubstanzen. Unter dieser Substanzklasse, werden sowohl organische als auch anorganische Gerüstsubstanzen verstanden. Es handelt sich dabei um Verbindungen, die sowohl eine Trägerfunktion in den erfindungsgemäßen Mitteln wahrnehmen können als auch bei der Anwendung als wasserenthärtende Substanz wirken.

Geeignete Builder sind beispielsweise Alkalimetallgluconate, -citrate, -nitrilotriacetate, -carbonate und -bicarbonate, insbesondere Natriumgluconat, -citrat und -nitrilotriacetat sowie Natrium- und Kaliumcarbonat und -bicarbonat, sowie Alkalimetall-und Erdalkalimetallhydroxide, insbesondere Natrium- und Kaliumhydroxid, Ammoniak und Amine, insbesondere Mono- und Triethanolamin, bzw. deren Mischungen. Hierzu zählen auch die Salze der Glutarsäure, Bernsteinsäure, Adipinsäure, Weinsäure und Benzolhexacarbonsäure sowie Phosphonate und Phosphate.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit, wie beispielsweise in den erfindungsgemäßen Granulaten, auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Die (co-)polymeren Polycarboxylate können entweder als Pulver oder als wäßrige Lösung eingesetzt werden. Der Gehalt der Mittel an (co-) polymeren Polycarboxylaten beträgt vorzugsweise 0,5 bis 20 Gew.-%, insbesondere 3 bis 10 Gew.-%.
Zur Verbesserung der Wasserlöslichkeit können die Polymere auch Allylsulfonsäuren, Allyloxybenzolsulfonsäure und Methallylsulfonsäure, als Monomer enthalten. Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder als Monomere Salze der Acrylsäure und der 2-Alkylallylsulfonsäure sowie Zucker-Derivate enthalten. Weitere bevorzugte Copolymere sind solche, die als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol. Ein bevorzugtes Dextrin ist in der britischen Patentanmeldung 94 19 091 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Co-builder. Dabei wird Ethylendiamin-N,N'-disuccinat (EDDS), dessen Synthese beispielsweise in US 3 158 615 beschrieben wird, bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Co-builder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Co-builder eingesetzt werden.

Ein bevorzugt eingesetzter anorganischer Builder ist feinkristalliner, synthetischer und gebundenes Wasser enthaltender Zeolith. Der eingesetzte feinkristalline, synthetische und gebundenes Wasser enthaltende Zeolith ist vorzugsweise Zeolith A und/oder P. Als Zeolith P wird beispielsweise Zeolith MAP z.B. Doucil A24^{®} (Handelsprodukt der Firma Crosfield) eingesetzt. Geeignet sind jedoch auch Zeolith X sowie Mischungen aus A, X und/oder P, beispielsweise ein Co-Kristallisat aus den Zeolithen A und X, der Vegobond^{®} AX (Handelsprodukt der Condea Augusta S.p.A.). Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser. In bevorzugten Ausführungsformen sind Zeolithe in Mengen von 10 bis 94,5 Gew.-% in dem Vorgemisch enthalten, wobei es kann besonders bevorzugt ist, wenn Zeolithe in Mengen von 20 bis 70, insbesondere 30 bis 60 Gew.-% enthalten sind.

Geeignete Teilsubstitute für Zeolithe sind Schichtsilicate natürlichen und synthetischen Ursprungs. Ihre Verwendbarkeit ist nicht auf eine spezielle Zusammensetzung bzw. Strukturformel beschränkt. Bevorzugt sind hier jedoch Smectite, insbesondere Bentonite. Auch kristalline, schichtförmige Natriumsilicate der allgemeinen Formel NaMSiₓO₂ₓ₊₁.yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind, eigenen sich zur Substitution von Zeolithen oder Phosphaten. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅.yH₂O bevorzugt.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Die Mittel enthalten Builder bevorzugt in Mengen, bezogen auf die Zusammensetzung, von 0 bis 20 Gew.-%, vorzugsweise 0,01 bis 12 Gew.-%, insbesondere 0,1 bis 8 Gew.-%, äußerst bevorzugt 0,3 bis 5 Gew.-%.

Neben den genannten Bestandteilen können die erfindungsgemäßen Wasch- und Reinigungsmittel zusätzlich einen oder mehrere Stoffe aus den Gruppen der Bleichmittel, Bleichaktivatoren, Enzyme, pH-Stellmittel, Fluoreszenzmittel, Farbstoffe, Schauminhibitoren, Silikonöle, Antiredepositionsmittel, optischen Aufheller, Vergrauungsinhibitoren, Farbübertragungsinhibitoren, Korrosionsinhibitoren und Silberschutzmittel enthalten. Diese Stoffe werden nachfolgend beschrieben.

Unter den als Bleichmittel dienenden, in Wasser H₂O₂ liefernden Verbindungen haben das Natriumperborattetrahydrat, das Natriumperboratmonohydrat und das Natriumpercarbonat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Auch beim Einsatz der Bleichmittel ist es möglich, auf den Einsatz von Tensiden und/oder Gerüststoffen zu verzichten, so dass reine Bleichmitteltabletten herstellbar sind. Sollen solche Bleichmitteltabletten zur Textilwäsche eingesetzt werden, ist eine Kombination von Natriumpercarbonat mit Natriumsesquicarbonat bevorzugt, unabhängig davon, welche weiteren Inhaltsstoffe in den Formkörpern enthalten sind. Werden Reinigungs-oder Bleichmitteltabletten für das maschinelle Geschirrspülen hergestellt, so können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesium-monoperphthalat, (b) die aliphatischen oder substituiert aliphatischen Peroxysäuren, wie Peroxylaurinsäure, Peroxystearinsäure, ε-Phthalimidoperoxycapronsäure [Phthaloiminoperoxyhexansäure (PAP)], o-Carboxybenzamidoperoxycapronsäure, N-nonenylamidoperadipin-säure und N-nonenylamidopersuccinate, und (c) aliphatische und araliphatische Peroxydi-carbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthaloyl-di(6-aminopercapronsäue) können eingesetzt werden.

Als Bleichmittel in Mitteln für das maschinelle Geschirrspülen können auch Chlor oder Brom freisetzende Substanzen eingesetzt werden. Unter den geeigneten Chlor oder Brom freisetzenden Materialien kommen beispielsweise heterocyclische N-Brom- und N-Chloramide, beispielsweise Trichlorisocyanursäure, Tribromisocyanursäure, Dibromisocyanursäure und/oder Dichlorisocyanur-säure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Um beim Waschen oder Reinigen bei Temperaturen von 60 °C und darunter eine verbesserte Bleichwirkung zu erreichen, können Bleichaktivatoren in die erfindungsgemäßen Wasch- und Reinigungsmittel eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n-bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze bzw. Übergangsmetallkomplexe wie beispielsweise Mn-, Fe-, Co-, Ru- oder Mo-Salenkomplexe oder -Carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase oder Protease und Cellulase oder aus Cellulase und Lipase oder aus Protease, Amylase und Lipase oder Protease, Lipase und Cellulase, insbesondere jedoch Cellulase-haltige Mischungen von besonderem Interesse. Auch Peroxidasen oder Oxidasen haben sich in einigen Fällen als geeignet erwiesen. Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate in den erfindungsgemäßen Formkörpern kann beispielsweise etwa 0,1 bis 5 Gew.-%, vorzugsweise 0,1 bis etwa 2 Gew.-% betragen. Zu den am häufigsten verwendeten Enzymen gehören Lipasen, Amylasen, Cellulasen und Proteasen. Bevorzugte Proteasen sind z.B. BLAP®140 der Fa. Biozym, Optimase®-M-440 und Opticlean®-M-250 der Fa. Solvay Enzymes; Maxacal®CX und Maxapem® oder Esperase® der Fa. Gist Brocades oder auch Savinase® der Fa. Novo. Besonders geeignete Cellulasen und Lipasen sind Celluzym® 0,7 T und Lipolase® 30 T der Fa. Novo Nordisk. Besondere Verwendung als Amylasen finden Duramyl® und Termamyl® 60 T, und Termamyl® 90 T der Fa. Novo, Amylase-LT® der Fa. Solvay Enzymes oder Maxamyl® P5000 der Fa. Gist Brocades. Auch andere Enzyme können verwendet werden.

Zusätzlich können die Wasch- und Reinigungsmittel auch Komponenten enthalten, welche die Öl- und Fettauswaschbarkeit aus Textilien positiv beeinflussen (sogenannte soil repellents). Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxy-propylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Die Mittel können als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)diphenyls, 4,4'-Bis(4-chlor-3-sulfostyryl)diphenyls, oder 4-(4-Chlorstyryl)-4'-(2-sulfostyryl)diphenyls. Auch Gemische der vorgenannten Aufheller können verwendet werden.

Um den ästhetischen Eindruck der erfindungsgemäßen Mittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Erfindungsgemäß sind Wasch- und reinigungsmittel auch Geschirrspülmittel. Die erfindungsgemäßen Geschirrspülmittel können zum Schutze des Spülgutes oder der Maschine Korrosionsinhibitoren enthalten, wobei besonders Silberschutzmittel im Bereich des maschinellen Geschirrspülens eine besondere Bedeutung haben. Allgemein können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders sauerstoff- und stickstoffhaltige organische redoxaktive Verbindungen, wie zwei- und dreiwertige Phenole, z.B. Hydrochinon, Brenzkatechin, Hydroxyhydrochinon, Gallussäure, Phloroglucin, Pyrogallol bzw. Derivate dieser Verbindungsklassen. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder-komplexe, besonders bevorzugt der Cobalt(ammin)-Komplexe, der Cobalt(acetat)-Komplexe, der Cobalt-(Carbonyl)-Komplexe, der Chloride des Cobalts oder Mangans und des Mangansulfats. Ebenfalls können Zinkverbindungen zur Verhinderung der Korrosion am Spülgut eingesetzt werden.

Besondere Inhaltsstoffe, die in erfindungsgemäßen Mitteln für das maschinelle Geschirrspülen oder die Reinigung harter Oberflächen genutzt werden können, sind Substanzen, die das Wiederanschmutzen von Oberflächen verhindern und/oder die Schmutzablösung nach einmaliger Anwendung erleichtern (sogenannte "Soil-Release-Verbindungen").
Zu den verwendbaren Soil-Release Verbindungen zählen alle im Stand der Technik bekannten Verbindungen. Besonders geeignet sind kationische Polymere, wie beispielsweise Hydroxypropyltrimethylammonium-Guar; Copolymere von Aminoethylmethacrylat und Acrylamid sowie Copolymere von Dimethyldiallylammoniumchlorid und Acrylamid, Polymere mit Imino-Gruppen, kationische Cellulosederivate, kationische Homo- und/oder Copolymere (Monomereinheiten: quaternisierte Ammoniumalkylmethacrylatgruppen).

Besonders bevorzugt sind die kationischen Polymeren ausgewählt aus kationischen Polymerisaten von Copolymeren von Monomeren wie Trialkylammoniumalkyl(meth)acrylat bzw. -acrylamid; Dialkyldiallyldiammoniumsalze; polymeranalogen Umsetzungsprodukten von Ethern oder Estern von Polysacchariden mit Ammoniumseitengruppen, insbesondere Guar-, Cellulose- und Stärkederivate; Polyaddukte von Ethylenoxid mit Ammoniumgruppen; quaternäre Ethyleniminpolymere und Polyester und Polyamide mit quaternären Seitengruppen als Soil-Release-Verbindungen. Außergewöhnlich bevorzugt im Rahmen dieser Anmeldung sind auch natürliche Polyuronsäuren und verwandte Substanzen, sowie Polyampholyte und hydrophobierte Polyampholyte, bzw. Gemische dieser Substanzen.

Diese Aufzählung von Wasch- und Reinigungsmittelinhaltsstoffen ist keineswegs abschließend, sondern gibt lediglich die wesentlichsten typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um ein- oder mehrwertige Alkohole mit 1 bis 4 C-Atomen. Bevorzugte Alkohole in solchen Mitteln sind Ethanol, 1,2-Propandiol, Glycerin sowie Gemische aus diesen Alkoholen. In bevorzugten Ausführungsformen enthalten derartige Mittel 2 bis 12 Gew.-% solcher Alkohole.

Grundsätzlich können die Mittel verschiedene Aggregatszustände aufweisen.
In einer weiteren bevorzugten Ausführungsform handelt es sich bei den Wasch- oder Reinigungsmitteln um flüssige oder gelförmige Mittel, insbesondere um Flüssigwaschmittel oder flüssige Geschirrspülmittel oder Reinigungsgele, wobei es sich insbesondere auch um gelförmige Reinigungsmittel für Spültoiletten handeln kann.
Es handelt sich dabei vorzugsweise um gelförmige, strukturviskose Reinigungsmittel mit einer Viskosität von 30000 - 150000 mPas, das als Gelbildner ein Polysaccharid, als Emulgator und netzaktive Komponente ein C₈₋₁₀-Alkylpolyglycosid oder C₁₂₋₁₄-Alkylpolyglycosid und Parfümöl enthalten. Als zusätzliche Co-Tenside können Fettalkoholethersulfate (FAEOS) und Fettalkoholsulfate (FAS) enthalten sein. Das Verhältnis APG zu Co-Tensid ist dann in der Regel größer als 1, vorzugsweise liegt es zwischen 50:1 und 1:1, besonders bevorzugt zwischen 10:1 und 1,5 zu 1 und ganz besonders bevorzugt zwischen 5:1 und 1,8:1. Insbesondere handelt es sich dabei um stabile, gelförmige, scherverdünnende Reinigungsmittel enthaltend Polysaccharid, ein Tensidsystem und Parfumkomponenten, die dadurch gekennzeichnet sind, dass
- ein Polysaccharid, bevorzugt ein Xanthan-Gum, in Mengen zwischen 1 und 5 Gew.-% bevorzugt 1 bis 4 Gew.-%, besonders bevorzugt 1,5 bis 3,5 Gew.-% und ganz besonders bevorzugt 1,8 bis 3 Gew.-% ,
- als eine Komponente des Tensidsystems ein C₈₋₂₂-Alkylpolyglycosid in Mengen zwischen 3 und 25 Gew.-% bevorzugt 4 und 20 Gew.-% besonders bevorzugt 5 und 15 Gew.-% und ganz besonders bevorzugt 5 und 12 Gew.-% und
- die Parfumkomponente oder die Parfumkomponenten bis 15 Gew.-% bevorzugt in 2 bis 12 Gew.-% besonders bevorzugt in 3 bis 8 Gew.-%
- sowie ggf. weitere Inhaltsstoffe wie kalklösende Mittel, Farbstoffe, keimhemmende Mittel (beispielsweise Isothiazolingemische, Natriumbenzoat oder Salicylsäure), Perlglanzmittel, Stabilisatoren Reinigungsverstärker und Geruchsabsorber enthalten sind
- und die Mittel eine Viskosität von 30000 bis 150000 mPas aufweisen, gemessen mit einem Brookfield Rotationsviskosimeter, Typ RVT mit Helipath-Einrichtung und der Spindel TA bei 1 U/min und 23 °C.

In den erfindungsgemäßen Gelen können ggf. wasserlösliche und wasserunlösliche Builder enthalten sein. Dabei sind dann wasserlösliche Builder bevorzugt, da sie auf harten Oberflächen in der Regel weniger dazu tendieren unlösliche Rückstände zu bilden. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildem zählen die Zeolithe, die ebenfalls verwendet werden können, ebenso wie Mischungen der vorgenannten Buildersubstanzen. Besonders bevorzugt ist die Gruppe der Citrate.

Weitere typische Reinigungsmittel, die die erfindungsgemäßen Kieselsäureester enthalten können, sind flüssige oder gelförmige Reiniger für harte Oberflächen, insbesondere sogenannte Allzweckreiniger, Glasreiniger, Boden- oder Badezimmerreiniger sowie spezielle Ausführungsformen derartiger Reiniger wozu saure oder alkalische Formen von Allzweckreinigern ebenso wie Glasreiniger mit sogenannter Anti-Rain-Wirkung gehören. Dabei können diese flüssigen Reinigungsmittel sowohl in einer als auch in mehreren Phasen vorliegen. In einer insbesondere bevorzugten Ausführungsform weisen die Reiniger 2 verschiedene Phasen auf.

Reiniger ist dabei im weitesten Sinne eine Bezeichnung für - zumeist Tensid-haltige - Formulierungen mit sehr weitem Einsatzbereich und davon abhängig sehr unterschiedlicher Zusammensetzung. Die wichtigsten Marktsegmente sind Haushalts-Reiniger, industrielle (technische) und institutionelle Reiniger. Nach dem pH-Wert unterscheidet man alkalische, neutrale und saure Reiniger, nach der Angebotsform flüssige und feste Reiniger (auch in Tablettenform). Die sogenannten Reiniger für harte Oberflächen sollen im Unterschied etwa zu Geschirrspülmitteln, die ebenfalls mit in die Produktgruppe der Reiniger eingeordnet werden, sowohl im konzentrierten Zustand als auch in verdünnter wäßriger Lösung. in Verbindung mit mechanischer Energie ein optimales Anwendungsprofil zeigen. Kaltreiniger entfalten ihre Leistung ohne erhöhte Temperatur. Maßgebend für die Reinigungswirkung sind v.a. Tenside und/oder Alkaliträger, alternativ Säuren, ggf. auch Lösungsmittel wie Glykolether und niedere Alkohole. Im Allgemeinen sind in den Formulierungen darüber hinaus Builder, je nach Reiniger-Typ auch Bleichmittel, Enzyme, keimmindernde oder desinfizierende Zusätze sowie Parfümöle und Farbstoffe enthalten. Reiniger können auch als Mikroemulsionen formuliert sein. Der Reinigungserfolg hängt in hohem Maße von der- auch geographisch sehr unterschiedlichen - Schmutzart und den Eigenschaften der zu reinigenden Oberflächen ab.

Haushalts-Reiniger: können als Universal-Reiniger oder als Spezial-Reiniger für u.a. Keramik, Fliesen, Fenster, Kunststoffe, (Teppich-)Böden, Kochfelder, Backöfen, Mikrowellenherde, als Sanitär-Reiniger oder WC-Reiniger formuliert werden. Rohr-Reiniger sind alkalisch eingestellt und bestehen z.B. aus festem Natriumhydroxid und Aluminium-Pulver, bei dessen Auflösung der entstehende Wasserstoff für eine entsprechende Verwirbelung in den freizuspülenden Rohrsegmenten sorgt. Sanitär-Reiniger enthalten neben Tensid und Builder v.a. keimmindernde Wirkstoffe, wobei das früher verwendete Natriumhypochlorit teilweise durch Wasserstoffperoxid oder andere Persauerstoff-Verbindungen ersetzt ist. WC-Reiniger sind überwiegend sauer, manchmal auch alkalisch eingestellt, wobei im ersteren Fall die ursprünglich verwendete Phosphorsäure und das Natriumhydrogensulfat weitgehend durch organische Säuren, v.a. Citronensäure, ersetzt sind. Zu den Spezial-Reinigern. gehören im Do-it-yourself-Bereich auch Kfz-, Autoscheiben-, Felgen-, Motoren- und Farbauftragsgeräte-Reiniger.

Institutionelle Reiniger: dienen der betrieblichen Reinigung und Hygiene z.B. in Schulen, Bürogebäuden, Hotels, Gaststätten und Krankenhäusern, wobei im letzteren Fall eine sichere Flächendesinfektion eine besondere Anforderung an die Produkte stellt. Diese Reiniger werden in Großgebinden abgegeben (Großverbraucherware). Die Produkte und die zugehörige Dienstleistung unter Einsatz speziell entwickelter Reinigungsgeräte werden als Systemlösung angeboten.

Technische Reiniger: Werden vor allem in der Getränke-, Nahrungsgüter-, kosmetischen und pharmazeutischen Industrie, aber auch in der Metall-Industrie zum Metallentfetten eingesetzt. Die Produktgruppe umfasst unter anderem auch Reiniger für Kfz-Waschanlagen, Tankwagen- und Flugzeug-Reiniger. Im Hinblick auf die erforderliche Produktivität z.B. bei der Flaschenreinigung müssen diese Reiniger mit schaumarmen Tensiden formuliert sein, wofür sich spezielle nichtionische Tenside, wie Ethylenoxid-Propylenoxid-Blockcopolymere und sogenannte endgruppenverschlossene Alkylethoxylate, eignen.

Die genannten Mittel können in einer besonders vorteilhaften Ausführungsform eine oder mehrere hydrophobe Komponenten enthalten. Geeignete Hydrophobkomponenten sind beispielsweise Dialkylether mit gleichen oder verschiedenen C₄₋₁₄-Alkylresten, insbesondere Dioctylether; Kohlenwasserstoffe mit einem Siedebereich von 100 bis 300 °C, insbesondere 140 bis 280 °C, z.B. aliphatische Kohlenwasserstoffe mit einem Siedebereich von 145 bis 200 °C, Isoparaffine mit einem Siedebereich von 200 bis 260 °C; etherische Öle, insbesondere Limonen und das aus Kiefernwurzeln und -stubben extrahierte Pine Oil; und auch Mischungen dieser Hydrophobkomponenten, insbesondere Mischungen von zwei oder drei der genannten Hydrophobkomponenten. Bevorzugte Gemische von Hydrophobkomponenten sind Gemische von verschiedenen Dialkylethern, von Dialkylethern und Kohlenwasserstoffen, von Dialkylethern und etherischen Ölen, von Kohlenwasserstoffen und etherischen Ölen, von Dialkylethern und Kohlenwasserstoffen und etherischen Ölen und von diesen Gemischen. Die Mittel enthalten Hydrophobkomponenten in Mengen, bezogen auf die Zusammensetzung, von 0 bis 20 Gew.-%, vorzugsweise 0,1 bis 14 Gew. %, insbesondere 0,5 bis 10 Gew.-%, äußerst bevorzugt 0,8 bis 7 Gew.-%.

Wegen ihrer schaumdämpfenden Eigenschaften können die Allzweckreiniger auch Seifen, d.h. Alkali- oder Ammoniumsalze gesättigter oder ungesättigter C₆₋₂₂-Fettsäuren, enthalten. Die Seifen können in einer Menge bis zu 5 Gew.-%, vorzugsweise von 0,1 bis 2 Gew.-%, eingesetzt werden.

Neben den genannten Komponenten können die erfindungsgemäßen Mittel weitere Hilfs- und Zusatzstoffe enthalten, wie sie in derartigen Mitteln üblich sind. Hierzu zählen insbesondere Polymere, Soil-Release-Wirkstoffe, Lösungsmittel (z.B. Ethanol, Isopropanol, Glykolether), Lösungsvermittler, Hydrotrope (z.B. Cumolsulfonat, Octylsulfat, Butylglucosid, Butylglykol), Reinigungsverstärker, Viskositätsregler (z.B. synthetische Polymere wie Polysaccharide, Polyacrylate, in der Natur vorkommenden Polymere und deren Derivate wie Xanthangum, weitere Polysaccharide und/oder Gelatine), pH-Regulatoren (z.B. Citronensäure, Alkanolamine oder NaOH), Desinfektionsmittel, Antistatika, Konservierungsmittel, Bleichsysteme, Enzyme, Farbstoffe sowie Trübungsmittel oder auch Hautschutzmittel.
Die Menge an derartigen Zusätzen liegt üblicherweise nicht über 12 Gew.-% im Reinigungsmittel. Die Untergrenze des Einsatzes hängt von der Art des Zusatzstoffes ab und kann beispielsweise bei Farbstoffen bis zu 0,001 Gew.-% und darunter betragen. Vorzugsweise liegt die Menge an Hilfsstoffen zwischen 0,01 und 7 Gew.-%, insbesondere 0,1 und 4 Gew.-%.

Die genannten Mittel können weiterhin Bindemittel enthalten, die allein oder in Mischung mit anderen Bindemitteln eingesetzt werden können. Bevorzugte Bindemittel sind Polyethylenglykole, 1,2-Polypropylenglykole sowie modifizierte Polyethylenglykole und Polypropylenglykole. Zu den modifizierten Polyalkylenglykolen zählen insbesondere die Sulfate und/oder die Disulfate von Polyethylenglykolen oder Polypropylenglykolen mit einer relativen Molekülmasse zwischen 600 und 12000 und insbesondere zwischen 1000 und 4000. Eine weitere Gruppe besteht aus Mono- und/oder Disuccinaten der Polyalkylenglykole, welche wiederum relative Molekülmassen zwischen 600 und 6000, vorzugsweise zwischen 1000 und 4000 aufweisen.
Im Rahmen dieser Erfindung zählen zu Polyethylenglykolen solche Polymere, bei deren Herstellung neben Ethylenglykol ebenso C₃-C₅-Glykole sowie Glycerin und Mischungen aus diesen als Startmoleküle eingesetzt werden. Ferner werden auch ethoxylierte Derivate wie Trimethylolpropan mit 5 bis 30 Ethylenoxid (EO) umfasst. Die vorzugsweise eingesetzten Polyethylenglykole können eine lineare oder verzweigte Struktur aufweisen, wobei insbesondere lineare Polyethylenglykole bevorzugt sind. Zu den insbesondere bevorzugten Polyethylenglykolen gehören solche mit relativen Molekülmassen zwischen 2000 und 12000, vorteilhafterweise um 4000, wobei Polyethylen-glykole mit relativen Molekülmassen unterhalb 3500 und oberhalb 5000 insbesondere in Kombination mit Polyethylenglykolen mit einer relativen Molekülmasse um 4000 eingesetzt werden können und derartige Kombinationen vorteilhafterweise zu mehr als 50 Gew.-%, bezogen auf die gesamte Menge der Polyethylenglykole, Polyethylenglykole mit einer relativen Molekülmasse zwischen 3500 und 5000 aufweisen. Als Bindemittel können jedoch auch Polyethylenglykole eingesetzt werden, welche an sich bei Raumtemperatur und einem Druck von 1 bar in flüssigem Stand vorliegen; hier ist vor allem von Polyethylenglykol mit einer relativen Molekülmasse von 200, 400 und 600 die Rede. Allerdings sollten diese an sich flüssigen Polyethylenglykole nur in einer Mischung mit mindestens einem weiteren Bindemittel eingesetzt werden, wobei diese Mischung wieder den erfindungsgemäßen Anforderungen genügen muss, also einen Schmelzpunkt bzw. Erweichungspunkt von mindestens oberhalb 45 °C aufweisen muss.

Ebenso eignen sich als Bindemittel niedermolekulare Polyvinylpyrrolidone und Derivate von diesen mit relativen Molekülmassen bis maximal 30000. Bevorzugt sind hierbei relative Molekülmassenbereiche zwischen 3000 und 30000, beispielsweise um 10000. Polyvinylpyrrolidone werden vorzugsweise nicht als alleinige Bindemittel, sondern in Kombination mit anderen, insbesondere in Kombination mit Polyethylenglykolen, eingesetzt.

Als geeignete weitere Bindemittel haben sich Rohstoffe erwiesen, welche Rohstoffe wasch-oder reinigungsaktiven Eigenschaften aufweisen, also beispielsweise nichtionische Tenside mit Schmelzpunkten von mindestens 45 °C oder Mischungen aus nichtionischen Tensiden und anderen Bindemitteln. Zu den bevorzugten nichtionischen Tensiden gehören alkoxylierte Fett- oder Oxoalkohole, insbesondere C₁₂₋₁₈-Alkohole. Dabei haben sich Alkoxylierungsgrade, insbesondere Ethoxylierungsgrade von durchschnittlich 18 bis 80 AO (Alkylenoxid), insbesondere Ethylenoxid (EO) pro Mol Alkohol und Mischungen aus diesen als besonders vorteilhaft erwiesen. Vor allem Fettalkohole mit durchschnittlich 18 bis 35 EO, insbesondere mit durchschnittlich 20 bis 25 EO, zeigen vorteilhafte Bindereigenschaften im Sinne der vorliegenden Erfindung. Gegebenenfalls können in Bindemittelmischungen auch ethoxylierte Alkohole mit durchschnittlich weniger EO-Einheiten pro Mol Alkohol enthalten sein, beispielsweise Talgfettalkohol mit 14 EO. Allerdings ist es bevorzugt, diese relativ niedrig ethoxylierten Alkohole nur in Mischung mit höher ethoxylierten Alkoholen einzusetzen. Vorteilhafterweise beträgt der Gehalt der Bindemittel an diesen relativ niedrig ethoxylierten Alkoholen weniger als 50 Gew.-%, insbesondere weniger als 40 Gew.-%, bezogen auf die Gesamtmenge an eingesetztem Bindemittel. Vor allem üblicherweise in Wasch- oder Reinigungsmitteln eingesetzte nichtionische Tenside wie C₁₂₋₁₈-Alkohole mit durchschnittlich 3 bis 7 EO, welche bei Raumtemperatur an sich flüssig vorliegen, sind vorzugsweise in den Bindemittelmischungen nur in den Mengen vorhanden, dass dadurch weniger als 2 Gew.-% dieser nichtionischen Tenside, bezogen auf das Verfahrensendprodukt, bereitgestellt werden.
Wie bereits oben beschrieben ist es allerdings weniger bevorzugt, in den Bindemittelmischungen bei Raumtemperatur flüssige nichtionische Tenside einzusetzen. In einer besonders vorteilhaften Ausführungsform sind derartige nichtionische Tenside aber kein Bestandteil der Bindemittelmischung, da diese nicht nur den Erweichungspunkt der Mischung herabsetzen, sondern auch zur Klebrigkeit des Endprodukts beitragen können und außerdem durch ihre Neigung, beim Kontakt mit Wasser zu Vergelungen zu führen, auch dem Erfordernis der schnellen Auflösung des Bindemittels/der Trennwand im Endprodukt nicht im gewünschten Umfang genügen. Ebenso ist es nicht bevorzugt, dass übliche in Wasch- oder Reinigungsmitteln eingesetzte Aniontenside oder deren Vorstufen, die Aniontensidsäuren, in der Bindemittelmischung enthalten sind. Andere nichtionische Tenside, die als Bindemittel geeignet sind, stellen die nicht zu Vergelungen neigenden Fettsäuremethylesterethoxylate, insbesondere solche mit durchschnittlich 10 bis 25 EO dar (genauere Beschreibung dieser Stoffgruppe siehe unten). Besonders bevorzugte Vertreter dieser Stoffgruppe sind überwiegend auf C₁₆₋₁₈-Fettsäuren basierende Methylester, beispielsweise gehärteter Rindertalgmethylester mit durchschnittlich 12 EO oder mit durchschnittlich 20 EO. In einer bevorzugten Ausführungsform der Erfindung wird als Bindemittel eine Mischung eingesetzt, welche C₁₂₋₁₈-Fettalkohol auf Basis Kokos oder Talg mit durchschnittlich 20 EO und Polyethylenglykol mit einer relativen Molekülmasse von 400 bis 4000 eingesetzt. In einer weiteren bevorzugten Ausführungsform der Erfindung wird als Bindemittel eine Mischung eingesetzt, welche überwiegend auf C₁₆₋₁₈-Fettsäuren basierende Methylester mit durchschnittlich 10 bis 25 EO, insbesondere gehärteten Rindertalgmethylester mit durchschnittlich 12 EO oder durchschnittlich 20 EO, und einem C₁₂₋₁₈-Fettalkohol auf Basis Kokos oder Talg mit durchschnittlich 20 EO und/oder Polyethylenglykol mit einer relativen Molekülmasse von 400 bis 4000 enthält.

Als besonders vorteilhafte Ausführungsformen der Erfindung haben sich Bindemittel erwiesen, die entweder allein auf Polyethylenglykolen mit einer relativen Molekülmasse um 4000 oder auf einer Mischung aus C₁₂₋₁₈-Fettalkohol auf Basis Kokos oder Talg mit durchschnittlich 20 EO und einem der oben beschriebenen Fettsäuremethylesterethoxylate oder auf einer Mischung aus C₁₂₋₁₈-Fettalkohol auf Basis Kokos oder Talg mit durchschnittlich 20 EO, einem der oben beschriebenen Fettsäuremethylesterethoxylate und einem Polyethylenglykol, insbesondere mit einer relativen Molekülmasse um 4000, basieren.

Als geeignete und altbekannte Desintegrationshilfsmittel können die erfindungsgemäßen Mittel beispielsweise Carbonat/Citronensäure-Systeme enthalten, wobei auch andere organische Säuren eingesetzt werden können. Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden im Rahmen der vorliegenden Erfindung Desintegrationsmittel auf Cellulosebasis eingesetzt, so dass bevorzugte Wasch- und Reinigungsmittelformkörper ein solches Desintegrationsmittel auf Cellulosebasis in Mengen von 0,5 bis 10 Gew.-%, vorzugsweise 3 bis 7 Gew.-% und insbesondere 4 bis 6 Gew.-% enthalten. Reine Cellulose weist die formale Bruttozusammensetzung (C₆H₁₀O₅)ₙ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50000 bis 500000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und - ether sowie Aminocellulosen. Die genannten Cellulosederivate werden vorzugsweise nicht allein als Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Desintegrationshilfsmittel eingesetzte Cellulose wird vorzugsweise nicht in feinteiliger Form eingesetzt, sondern vor dem Zumischen zu den zu verpressenden Vorgemischen in eine gröbere Form überführt, beispielsweise granuliert oder kompaktiert.

Die Teilchengrößen solcher Desintegrationsmittel liegen zumeist oberhalb 200 µm, vorzugsweise zu mindestens 90 Gew.-% zwischen 300 und 1600 µm und insbesondere zu mindestens 90 Gew.-% zwischen 400 und 1200 µm.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind.

In einer bevorzugten Variante enthalten die Wasch- und Reinigungsmittel, insbesondere in Form von Formkörper wie Tabletten 0,5 bis 10 Gew.-%, vorzugsweise 3 bis 7 Gew.-% und insbesondere 4 bis 6 Gew.-% eines oder mehrerer Desintegrationshilfsmittel, jeweils bezogen auf das Förmkörpergewicht.

Ein weiterer Gegenstand der vorliegenden Erfindung sind kosmetische Mittel bzw. zur Haar-oder Hautbehandlung, enthaltend die erfindungsgemäßen Kieselsäureester.

Vorzugsweise enthalten diese kosmetischen Mittel die erfindungsgemäßen Kieselsäureester in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf die Gesamtzusammensetzung des kosmetischen Mittels.
Die Gesamtmenge der Duftstoffe in den kosmetsichen Mittel beträgt dagegen vorzugsweise zwischen 0,01 und 5 Gew.-%, besonders vorzugsweise zwischen 0,1 und 3 Gew.-% sowie ganz besonders bevorzugt zwischen 0,5 und 2 Gew.-% bezogen auf die Gesamtmenge des Mittels. Bevorzugt werden Mischungen verschiedener Duftstoffe (aus den verschiedenen oben genannten Duftstoffklassen) verwendet, die gemeinsam eine ansprechende Duftnote erzeugen. In diesem Fall ist die Gesamtmenge des mindestens einen Duftstoffs die Menge aller Duftstoffe in der Mischung zusammen bezogen auf die Gesamtmenge des Mittels.

In einer bevorzugten Ausführungsform handelt es sich bei den kosmetischen Mitteln um wässrige Zubereitungen, die oberflächenaktive Wirkstoffe enthalten und die sich insbesondere zur Behandlung von Keratinfasern, insbesondere menschlichen Haaren, oder zur Behandlung von Haut eignen.

Bei den angesprochenen Haarbehandlungsmitteln handelt es sich dabei insbesondere um Mittel zur Behandlung von menschlichem Kopfhaar. Die gebräuchlichsten Mittel dieser Kategorie lassen sich einteilen in Haarwaschmittel, Haarpflegemittel, Haarverfestigungs- und Haarverformungsmittel sowie Haarfärbemittel und Haarentfemungsmittel. Zu den erfindungsgemäß bevorzugten, oberflächenaktive Wirkstoffe enthaltenden Mittel zählen insbesondere Haarwasch- und Pflegemittel: Ein derartiges Haarwaschmittel oder Haarshampoo besteht aus 10 bis 20, in Einzelfällen bis zu 30 Rezepturbestandteilen. Diese wässrigen Zubereitungen liegen meist in flüssig bis pastöser Form vor.
Die erfindungsgemäßen kosmetischen Mittel, enthalten in der Regel noch weitere Inhaltsstoffe, die für diese Mittel üblich sind.

Vorzugsweise enthalten die erfindungsgemäßen kosmetischen Mittel oberflächenaktiven Wirkstoffe oder Waschaktivstoffe als weitere Inhaltsstoffe.
Bevorzugt werden hierbei Fettalkoholpolyglykolethersulfate (Ethersulfate, Alkylethersulfate) eingesetzt, zum Teil in Kombination mit anderen meist anionischen Tensiden.
Neben den Alkylethersulfaten können bevorzugte Mittel zusätzlich weitere Tenside, wie Alkylsulfate, Alkylethercarboxylate, vorzugsweise mit Ethoxylierungsgraden von 4 bis 10, sowie tensidische Eiweiß-Fettsäure-Kondensate enthalten. Hier ist insbesondere das Eiweiß-Abitinsäure-Kondensat zu erwähnen. Auch Sulfobernsteinsäureester, Amidopropyplbetaine, Amphoacetate und Amphodiacetate sowie Alkylpolyglykoside sind in Haarshampoos bevorzugt eingesetzte Tenside.

Eine weitere Gruppe von Inhaltsstoffen wird unter dem Begriff Hilfsstoffe zusammengefasst und ist sehr vielfältig: z.B. erhöhen Zusätze von nichtionischen Tensiden wie ethoxylierten Sorbitanestern oder von Eiweiß-Hydrolysaten die Verträglichkeit bzw. wirken reizmindemd, z.B. in Baby-Shampoos; als Rückfetter zur Vorbeugung zu starker Entfettung bei der Haarwäsche dienen z.B. natürliche Öle oder synthetische Fettsäureester; als Feuchthaltemittel dienen Glycerin, Sorbit, Propylenglykol (s. Propandiole), Polyethylenglykole u.a. Polyole. Zur Verbesserung der Naßkämmbarkeit und Verminderung elektrostatischer Aufladung der Haare nach dem Trocknen können den Shampoos Kationtenside wie z.B. quartäre Ammonium-Verbindungen zugesetzt werden. Für ein farbiges, brillantes Erscheinungsbild werden Farbstoffe bzw. Perlglanzpigmente zugesetzt. Zur Einstellung der gewünschten Viskosität können Verdickungsmittel verschiedener Stoffklassen verwendet werden, eine pH-Stabilität wird durch Puffer z.B. auf der Basis von Citrat, Lactat oder Phosphat erzielt. Um eine ausreichende Haltbarkeit und Lagerfähigkeit zu gewährleisten, werden Konservierungsmittel wie z.B. 4-Hydroxybenzoesäureester zugesetzt; oxidationsempfindliche Inhaltsstoffe können durch Zusatz von Antioxidantien wie Ascorbinsäure, Butylmethoxyphenol oder Tocopherol geschützt werden.

Eine weitere bevorzugte Gruppe von Inhaltsstoffen bilden spezielle Wirkstoffe für Spezial-Shampoos, z.B. Öle, Kräuterextrakte, Proteine, Vitamine und Lecithine in Shampoos für schnell fettendes, für besonders trockenes, für strapaziertes oder geschädigtes Haar. Wirkstoffe in Shampoos zur Bekämpfung von Schuppen haben meist eine breite wachstumshemmende Wirkung gegen Pilze und Bakterien. Insbesondere die fungistatischen Eigenschaften z.B. von Pyrithion-Salzen konnten als Ursache guter Antischuppen-Wirkung nachgewiesen werden. Zur Erzeugung einer angenehmen Duftnote enthalten die Haarshampoos Parfümöle. Dabei können die Shampoos ausschließlich die erfindungsgemäßen Kieselsäureester enthalten, es ist jedoch ebenfalls bevorzugt wenn die Haarshampoos nicht nur diese, sondern auch andere Duftstoffe daneben enthalten. Dabei können alle üblichen, und in Haarshampoos zugelassenen Duftstoffe eingesetzt werden.

Haarpflegemittel haben zum Ziel den Naturzustand des frisch nachgewachsenen Haares möglichst lange zu erhalten und bei Schädigung wiederherzustellen. Merkmale die diesen Naturzustand charakterisieren sind seidiger Glanz, geringe Porosität, spannkräftige und dabei weiche Fülle und angenehm glattes Gefühl. Eine wichtige Voraussetzung hierfür ist eine saubere, schuppenfreie und nicht überfettete Kopfhaut. Zu den Haarpflegemitteln zählt man heute eine Vielzahl verschiedener Produkte, deren wichtigste Vertreter als Vorbehandlungsmittel, Haarwässer, Frisierhilfsmittel, Haarspülungen und Kurpackungen bezeichnet werden, und deren Zusammensetzung wie bei den Haarwaschmitteln grob in Grundstoffe, Hilfsstoffe und spezielle Wirkstoffe aufgegliedert wird.

Als Grundstoffe dienen Fettalkohole, v.a. Cetylalkohol (1-Hexadecanol) und Stearylalkohol (1-Octadecanol), Wachse wie Bienenwachs, Wollwachs (Lanolin), Walrat und synthetische Wachse, Paraffine, Vaseline, Paraffinöl sowie als Lösungsmittel. v.a. Ethanol, 2-Propanol und Wasser. Hilfsstoffe sind Emulgatoren, Verdickungsmittel, Konservierungsmittel, Antioxidantien, Farbstoffe und Parfüm-Öle. Die heute wichtigste Gruppe spezieller Wirkstoffe in den Haarpflegemitteln sind die quartären Ammonium-Verbindungen. Man unterscheidet monomere (z.B.: Alkyltrimethylammoniumhalogenid mit v.a. der Lauryl-, Cetyl- oder Stearyl-Gruppe als Alkyl-Rest) und polymere quartäre Ammonium-Verbindungen [z.B.: quartäre Celluloseether-Derivate oder Poly(N,N-dimethyl-3,4-methylenpyrrolidiniumchlorid)]. Ihre Wirkung in Haarpflegemitteln beruht darauf, daß die positive Ladung der Stickstoff-Atome dieser Verbindung sich an die negativen Ladungen des Haar-Keratins anlagern kann; geschädigte Haare enthalten wegen ihres höheren Cysteinsäure-Gehalts mehr negativ geladene Säure-Gruppen und können daher mehr quartäre Ammonium-Verbindungen aufnehmen. Diese, wegen ihres kationaktiven Charakters auch als "kationaktive Pflegestoffe" bezeichnet, wirken glättend auf das Haar, verbessern die Kämmbarkeit, vermindern die elektrostatische Aufladung, verbessern Griff und Glanz. Die polymeren quartären Ammonium-Verbindungen haften so gut am Haar, daß ihre Wirkung noch nach mehreren Wäschen nachgewiesen werden kann. Organische Säuren wie Citronensäure, Weinsäure oder Milchsäure werden häufig zur Einstellung eines sauren Milieus eingesetzt. Die wasserlöslichen Eiweiß-Hydrolysate ziehen wegen ihrer engen chemischen Verwandtschaft gut auf das Haar-Keratin auf.
Die größte Gruppe spezieller Wirkstoffe in Haarpflegemitteln bilden diverse Pflanzenextrakte und Pflanzenöle.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.
Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Ginseng und Ingwerwurzel.
Ganz besonders geeignet sind die Extrakte aus Grünem Tee, Mandel, Aloe Vera, Kokosnuss, Mango, Aprikose, Limone, Weizen, Kiwi und Melone.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Weiterhin kann es bevorzugt sein, in den erfindungsgemäßen Mitteln Mischungen aus mehreren, insbesondere aus zwei, verschiedenen Pflanzenextrakten einzusetzen.

Zur Vermeidung einer zu schnellen Rückfettung enthalten einige Haarwässer Substanzen wie gewisse Teer-Inhaltsstoffe, Cysteinsäure-Derivate oder Glycyrrhizin; die beabsichtigte Verminderung der Talgdrüsenproduktion ist ebenfalls noch nicht eindeutig bewiesen. Dagegen ist die Wirksamkeit von Antischuppen-Wirkstoffen einwandfrei belegt. Sie werden daher in entsprechenden Haarwässem u.a. Pflegemitteln eingesetzt.

Bei den wässrigen Zubereitungen zur Behandlung von Haut handelt es sich insbesondere um Zubereitungen zur Pflege menschlicher Haut. Diese Pflege beginnt mit der Reinigung für die in erster Linie Seifen benutzt werden. Hier unterscheidet man feste, meist stückige und flüssige Seife. Dementsprechend liegen die kosmetischen Mittel in einer bevorzugten Ausführungsform als Formkörper vor, die oberflächenaktive Inhaltsstoffe enthalten. Wichtigste Inhaltsstoffe derartiger Formkörper sind in einer bevorzugten Ausführungsform die Alkalisalze der Fettsäuren natürlicher Öle und Fette, vorzugsweise mit Ketten von 12-18 C-Atomen. Da Laurinsäure-Seifen besonders gut schäumen, sind die Laurinsäure-reichen Kokos- und Palmkem-Öle bevorzugte Rohstoffe für die Feinseifen-Herstellung. Die Na-Salze der Fettsäure-Gemische sind fest, die K-Salze weich-pastös. Zur Verseifung wird die verdünnte Natron- oder Kali-Lauge den Fett-Rohstoffen im stöchiometrichem Verhältnis so zugesetzt, daß in der fertigen Seife ein Laugenüberschuß von max. 0,05% vorhanden ist. Vielfach werden die Seifen heute nicht mehr direkt aus den Fetten, sondern aus den durch Fettspaltung gewonnenen Fettsäuren hergestellt. Übliche Seifen-Zusätze sind Fettsäuren, Fettalkohole, Lanolin, Lecithin, pflanzliche Öle, Partialglyceride u.a. fettähnliche Substanzen zur Rückfettung der gereinigten Haut, Antioxidantien wie Ascorbyl-Palmitat oder Tocopherol zur Verhinderung der Autoxidation der Seife (Ranzigkeit), Komplexierungsmittel wie Nitrilotriacetat zur Bindung von Schwermetall-Spuren, die den autoxidativen Verderb katalysieren könnten, Parfüm-Öle zur Erzielung der gewünschten Duftnoten, Farbstoffe zur Einfärbung der Seifenstücke und ggf. spezielle Zusätze.

Flüssige Seifen basieren sowohl auf K-Salzen natürlicher Fettsäuren als auch auf synthetischen Aniontensiden. Sie enthalten in wässriger Lösung weniger waschaktive Substanzen als feste Seifen, haben die üblichen Zusätze, ggf. mit viskositätsregulierenden Bestandteilen sowie Perlglanz-Additiven. Wegen ihrer bequemen und hygienischen Anwendung aus Spendern werden sie vorzugsweise in öffentlichen Waschräumen und dergleichen verwendet. Wasch-Lotionen für besonders empfindliche Haut basieren auf mild wirkenden synthetischen Tensiden mit Zusätzen hautpflegender Substanzen, pH-neutral oder schwach sauer (pH 5,5) eingestellt.

Zur Reinigung und Pflege vornehmlich der Gesichtshaut gibt es eine Reihe weitere Präparate, wie Gesichtswässer, Reinigungs-Lotionen, -Milche, -Cremes, -Pasten; Gesichtspackungen dienen z.T. der Reinigung, überwiegend jedoch der Erfrischung und Pflege der Gesichtshaut. Gesichtswässer sind meist wässrige-alkoholische Lösungen mit geringen Tensid-Anteilen sowie weiteren hautpflegenden Substanzen. Reinigungs-Lotionen, -Milche, -Cremes und - Pasten basieren meist auf O/W-Emulsionen mit relativ geringen Gehalten an Fettkomponenten mit reinigenden und pflegenden Zusätzen. Sogenannte Scruffing- und Peeling-Präparate enthalten mild keratolytisch wirkende Substanzen zur Entfernung der obersten abgestorbenen Haut-Horn-Schichten, z.T. mit Zusätzen abrasiv wirkender Pulver.
In Mitteln zur reinigenden Behandlung unreiner Haut sind außerdem antibakterielle und entzündungshemmende Substanzen enthalten, da die Talgansammlungen in Komedonen (Mitessern) Nährböden für bakterielle Infektionen darstellen und zu Entzündungen neigen.

Die angebotene breite Palette verschiedener Hautreinigungs-Produkte variiert in Zusammensetzung und Gehalt an diversen Wirkstoffen, abgestimmt auf die verschiedenen Hauttypen und auf spezielle Behandlungsziele.

Die für die Hautreinigung im Wannen- oder Duschbad angebotenen Badezusätze haben breite Anwendung gefunden. Badesalze und Badetabletten sollen das Badewasser enthärten, färben und parfümieren und enthalten in der Regel keine waschaktiven Substanzen. Durch die Enthärtung des Badewassers fördern sie die Reinigungskraft von Seifen, sollen jedoch in erster Linie erfrischend wirken und das Badeerlebnis verstärken. Größere Bedeutung haben die Schaumbäder. Bei einem höheren Gehalt an rückfettenden und hautpflegenden Substanzen spricht man auch von Creme-Bädern.

Die erfindungsgemäßen kosmetischen Mittel können in unterschiedlichen Zubereitungsformen vorliegen.
Die wichtigsten sind Haut-Cremes, -Lotionen, -Öle und -Gele. Basis der Cremes und Lotionen sind Emulsionen in O/W- (Öl in Wasser) od. W/O- (Wasser in Öl) Form. Die Hauptbestandteile der Öl- bzw. Fett- oder Lipid-Phase sind Fettalkohole, Fettsäuren, Fettsäureester, Wachse, Vaseline, Paraffine sowie weitere Fett- und Ölkomponenten hauptsächlich natürlichen Ursprungs. In der wässrigen Phase sind neben Wasser hauptsächlich feuchtigkeitsregulierende und feuchtigkeitsbewahrende Substanzen als wesentliche Hautpflege-Wirkstoffe enthalten, ferner konsistenz- bzw. viskositätsregulierende Mittel. Weitere Zusätze wie Konservierungsmittel, Antioxidantien, Komplexbildner, ParfümÖle, Färbemittel sowie spezielle Wirkstoffe werden je nach ihrer Löslichkeit und ihren Stabilitätseigenschaften einer der beiden vorgenannten Phasen beigegeben. Wesentlich für den Emulsionstyp und seine Eigenschaften ist die Auswahl des Emulgator-Systems. Seine Auswahl kann nach dem HLB-System erfolgen.

Weiterhin können die Hautpflegemittel weitere spezielle Wirkstoffe wie beispielsweise Milcheiweißprodukte, Eigelb, Lecithine, Lipoide, Phosphatide, Getreidekeimöle, Vitamine - insbesondere Vitamin F und das früher als Hautvitamin (Vitamin H) bezeichnete Biotin sowie hormonfreie Placenta-Extrakte, enthalten.

Hautöle gehören zu den ältesten Produktformen der Hautpflege und werden noch heute verwendet. Basis sind nichttrocknende Pflanzenöle wie Mandelöl oder Olivenöl, mit Zusätzen natürlicher Vitaminöle wie Weizenkeimöl oder Avocadoöl sowie öligen Pflanzenextrakten aus z.B. Johanniskraut, Kamille u.ä..
Hautgele sind halbfeste transparente Produkte, die durch entsprechende Gelbildner stabilisiert werden. Man unterscheidet Oleogele (wasserfrei), Hydrogele (ölfrei) und Öl/Wasser-Gele. Die Typenauswahl richtet sich nach dem gewünschten Anwendungs-Zweck. Die Öl/Wasser-Gele enthalten hohe Emulgator-Anteile und weisen gegenüber Emulsionen gewisse Vorteile auf sowohl unter ästhetischen als auch unter Anwendungsgesichtspunkten.

Weitere erfindungsgemäß bevorzugte kosmetische Mittel sind Mittel zur Beeinflussung des Körpergeruchs. Insbesondere sind hier deodorierende Mittel gemeint. Derartige Deodorantien können Gerüche überdecken, entfernen oder zerstören. Unangenehme Körpergerüche entstehen bei bakterieller Zersetzung des Schweißes, insbesondere in den feuchtwarmen Achselhöhlen, wo Mikroorganismen gute Lebensbedingungen finden. Dementsprechend sind die wichtigsten Inhaltsstoffe von Deodorantien keimhemmende Substanzen. Insbesondere sind solche keimhemmenden Substanzen bevorzugt, die eine weitgehende selektive Wirksamkeit gegenüber den für den Körpergeruch verantwortlichen Bakterien besitzen. Bevorzugte Wirkstoffe haben dabei jedoch lediglich eine bakteriostatische Wirkung und töten die Bakterienflora keinesfalls ganz ab. Zu dem keimhemmenden Mitteln können generell alle geeigneten Konservierungsmittel mit spezifischer Wirkung gegen grampositiver Bakterien gerichtet werden. Beispielsweise sind dies Irgasan DP 300 (Trichlosan, 2,4,4'-Trichlor-2'-Hydroxydiphenylether), Chlorhexidin (1,1'-Hexamethylenbis(5-(4'-chlor-phenyl)-biguanid) sowie 3,4,4'-Trichlorcarbanilid. Auch quartäre Ammonium-Verbindungen sind prinzipiell ebenfalls geeignet. Aufgrund ihrer hohen antimikrobierenden Wirksamkeit werden all diese Stoffe bevorzugt nur in geringen Konzentrationen von etwa 0,1 bis 0,3 Gew.-% eingesetzt. Weiterhin haben auch zahlreiche Riechstoffe antimikrobielle Eigenschaften. Dementsprechend werden derartige Riechstoffe mit antimikrobiellen Eigenschaften in Deodorantien bevorzugt eingesetzt. Insbesondere sind hier Farnesol und Phenoxyethanol zu nennen. Daher ist es insbesondere bevorzugt, wenn die erfindungsgemäßen Deodorantien solche selbst bakteriostatisch wirksamen Riechstoffe enthalten. Dabei können die Riechstoffe vorzugsweise wieder in Form von Kieselsäureestern enthalten sein. Es ist jedoch auch möglich, daß gerade diese antibakteriell wirksamen Riechstoffe nicht in Form von Kieselsäureestern eingesetzt werden und dann in Mischungen mit anderen Riechstoffen, die als Kieselsäureester vorliegen, eingesetzt sind. Eine weitere Gruppe wesentlicher Inhaltsstoffe von Deodorantien sind Enzyminhibitoren, die die Zersetzung des Schweißes durch Enzyme hemmen, wie beispielsweise Citronensäuretriethylester oder Zinkglycinat. Wesentliche Inhaltsstoffe von Deodorantien sind weiterhin auch Antioxidantien, die eine Oxidation der Schweißbestandteile verhindern sollen.

Ein weiterer Gegenstand der vorliegenden Erfindung ist die Verwendung der erfindungsgemäßen Kieselsäureester zur Verlängerung der Duftwirkung von Duftstoffen.
Aufgrund der hervorragenden Eignung der erfindungsgemäßen Verbindungen zum Einsatz in Wasch- und Reinigungsmitteln ist die Verwendung der erfindungsgemäßen Kieselsäureester, in flüssigen oder festen Wasch- und Reinigungsmitteln, insbesondere bevorzugt als Duftstoff, ein weiterer Gegenstand der vorliegenden Erfindung.
Ebenso eignen sich die erfindungsgemäßen Kieselsäureester hervorragend zum Einsatz in kosmetischen Mitteln, daher ist ein weiterer Gegenstand der vorliegenden Erfindung die Verwendung der erfindungsgemäßen Kieselsäureester in kosmetischen Mitteln zur Haut- und Haarbehandlung insbesondere bevorzugt als Duftstoff.
Bevorzugt werden dabei Kieselsäureester verwendet, die als Duftstoffreste Duftstoffaldehyde oder Duftstoffketone aufweisen.
Die erfindungsgemäßen Kieselsäureester werden vorzugsweise zusammen mit anderen Duftstoffen eingesetzt. Dabei können die Duftstoffe vorzugsweise wieder in Form von Kieselsäureestern oder auch in Mischungen mit anderen Duftstoffen, die keine Profragrances sind, vorliegen.
Bevorzugt werden die erfindungsgemäßen Kieselsäureester in den genannten Einsatzgebieten in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das Mittel, eingesetzt .

Ein weiterer Gegenstand der vorliegenden Erfindung ist das Verfahren zur Verlängerung des Duftempfindens von Wasch- oder Reinigungsmitteln, Weichspülern oder Kosmetika oder mit diesen behandelten festen Oberflächen, dadurch gekennzeichnet, dass man die erfindungsgemäßen Kieselsäureester oder Mischungen daraus zu den Wasch- oder Reinigungsmitteln, Weichspülern oder Kosmetika zugibt.
Die Duftstoffe, die an den erfindungsgemäßen Kieselsäureestern gebunden vorliegen, werden dabei bevorzugt nach und nach durch Hydrolyse wieder freigesetzt.

Die erfindungsgemäßen Kieselsäureester können als alleiniger Duftstoff eingesetzt werden, es ist aber auch möglich, Duftstoffgemische einzusetzen, die lediglich zu einem Teil aus den erfindungsgemäßen Kieselsäureestern bestehen. Solche Gemische haben den Vorteil, daß auch die Bestandteile des Duftstoffgemischs, die nicht in Form von Kieselsäureester vorliegen, in der Haltbarkeit des Dufteindrucks verbessert werden. So können insbesondere Duftstoffgemische eingesetzt werden, die 1 bis 50 Gew.-%, vorzugsweise 5 bis 40 und insbesondere maximal 30 Gew.-% an Kieselsäureester enthalten. In anderen Ausführungsformen, bei denen insbesondere die verzögerte Duftwirkung der Trägergebundenen Form genutzt werden soll, werden bei der erfindungsgemäßen Verwendung vorteilhaft mindestens 30 Gew.-%, vorzugsweise mindestens 40 Gew.-% und insbesondere mindestens 50 Gew.-% des insgesamt im Mittel enthaltenen Parfüms über die erfindungsgemäßen Kieselsäureester in die Mittel eingebracht, während die restlichen 70 Gew.-%, vorzugsweise 60 Gew.-% und insbesondere 50 Gew.-% des insgesamt im Mittel enthaltenen Parfüms auf übliche Weise aufgesprüht oder anders in die Mittel eingebracht werden. Die erfindungsgemäße Verwendung kann also vorteilhaft dadurch gekennzeichnet sein, daß die Kieselsäureester zusammen mit anderen Duftstoffen eingesetzt werden.

Die Erfindung wird durch die nachstehenden Beispiele näher erläutert.

### Beispiele

### 1. Synthese von 1-Aza-3,7-dioxa-bicyclo[3.3.0]octan und/oder 1,3-Oxazolidin-Derivaten

### AA1: Allgemeine Arbeitsvorschrift zur Synthese von 1-Aza-3,7-dioxa-bicyclo[3.3.0]octanund/oder 1,3-Oxazolidin-Derivaten, Verhältnis Aminoalkohol/Duftstoffaldehyd bzw. -keton

Der Aminoalkohol wird unter Stickstoffatmosphäre mit dem Aldehyd bzw. Keton im Verhältnis 1:2 (1-Aza-3,7-dioxa-bicyclo[3.3.0]octan-Derivate) bzw. 1:1 (1,3-Oxazolidin-Derivate) in Toluol vorgelegt. Das Reaktionsgemisch wird auf T = 120 °C erhitzt, wobei der Aminoalkohol langsam in Lösung geht. Rückflussen am Wasserabscheider für 7h. Einrotieren und Trocknen der klaren leicht gelblichen Lösung im Hochvakuum.

### AA2: Allgemeine Arbeitsvorschrift zur Synthese von 1-Aza-3,7-dioxa-bicyclo[3.3.0]octan-und/oder 1,3-Oxazolidin- Derivaten, Verhältnis Aminoalkohol/DuftstoffAldehyd bzw. -keton in situ

Der Aminoalkohol wird unter Stickstoffatmosphäre mit dem Aldehyd bzw. Keton im Verhältnis 1:2 (1-Aza-3,7-dioxa-bicyclo[3.3.0]octan-Derivate) bzw. 1:1 (1,3-Oxazolidin-Derivate) vorgelegt. Das Reaktionsgemisch wird auf T = 100 °C-140°C erhitzt, wobei die Reaktanden langsam in Lösung gehen oder schmelzen. Das Reaktionsgemisch wird so lange erhitzt, bis kein Reaktionswasser mehr abdestilliert werden kann. Trocknen der klaren leicht gelblichen Lösung im Hochvakuum.
Eine weitere Aufreinigung der entsprechenden 1-Aza-3,7-dioxa-bicyclo[3.3.0]octan- und/oder 1,3-Oxazolidin-Derivate kann säulenchromatographisch mit Kieselgel als stationärer Phase erfolgen.
Beispielsweise gelingt bei Raumtemperatur die Aufreinigung von Phenyl-1-methylenheptyl-1,3-oxazolidin-4-yl-methanol mit Kieselgel als stationärer Phase und einer Toluol/Ethylacetat 15:1-Mischung als Eluenten. Der Rf-Wert der oben genannten Substanz beträgt dabei 0,09.

### 2. Synthese der erfindungsgemäßen Kieselsäureester

### 2.1. AA3: Umesterung von AA1 oder AA2 zum Kieselsäureester

Die Herstellung der erfindungsgemäßen Kieselsäureester gelingt durch einfache Umesterung von Oligokieselsäureestern niederer Alkohole mit den Verbindungen, die aus AA1 oder AA2 erhalten wurden.

Je nach Reaktionszeit und -bedingungen werden die niederen Alkohole abgespalten und die Verbindungen aus AA1 bzw. AA2 gebunden, wobei die Alkohole entlang von Si-O-Si-Ketten oder -Ringen leichter ausgetauscht werden als die terminalen Alkohole.
Derartige Umesterungen lassen sich beispielsweise, wie in der Publikation H. Steinmann, G. Tschernko, H. Hamann, Z. Chem. 3, 1977, S.89-92 beschrieben, durchführen.
Üblicherweise werden als Edukte die handelsüblichen Kieselsäureester eingesetzt. Hier ist insbesondere der Ethanol-Ester zu nennen, der beispielsweise bei der Fa. Wacker, Burghausen erhalten werden kann. Die Umesterung kann dabei ausschließlich durch Temperaturerhöhung und Abdestillation der leichtflüchtigen Nebenprodukte gesteuert werden. Bevorzugt ist es jedoch, wenn zur Umesterung Katalysatoren eingesetzt werden. Es handelt sich dabei üblicherweise um Lewis-Säuren, vorzugsweise um Aluminiumtriisopropylat, Titantetraisopropylat, Siliciumtetrachlorid oder basische Katalysatoren oder auch um Zubereitungen wie beispielsweise aus Aluminiumoxid mit Kaliumfluorid.

### 2.2. AA4: Polymeranaloge Umsetzung zum Kieselsäureester

Der Aminoalkohol kann auch direkt mit teilhydrolysiertem Tetraethoxysilan unter Durchleitung von Stickstoff bei 120 °C umgesetzt werden, wobei Ethanol abgespalten wird. Anschließend wird abgekühlt und der Aldehyd bzw. das Keton zugegeben und erneut bis auf 110 °C erhitzt. Das Reaktionswasser wird im Hochvakuum bei 110°C abdestilliert. Nach ca 4 h Reaktionszeit wird das Reaktionsprodukt auf Raumtemperatur abgekühlt und eine klare, hochviskose Flüssigkeit erhalten.

Der Reaktionsfortschritt der Herstellung der erfindungsgemäßen Kieselsäureester gemäß AA3 oder AA4 kann leicht über GC-Analytik verfolgt werden. Dabei wird in regelmäßigen Abständen aus der Reaktionslösung eine Probe entnommen, in der per GC-Analytik der Gehalt an verbliebenen 1-Aza-3,7-dioxa-bicyclo[3.3.0]octan- und/oder 1,3-Oxazolidin-Derivaten bestimmt wird.
Beispielsweise kann die Umsetzung von 1-Aza-3,7-dioxa-2,8-diheptyl-5-methyloxybicyclo[3.3.0]octan mit folgender GC-Analytik verfolgt werden:

| | |
|---|---|
| GC- Gerät: | Hewlett Packard 5890 Series II |
| Säule : | HP 1 Crosslinked Methylsilicone Gum (25 m x 0,32 mm x 0,17 µ m) |
| Starttemperatur : | 45 °C |
| Heizrate : | 15 ° C/min |
| Endtemperatur : | 300 °C |
| Final-Time : | 5 min |
| Retentionszeit 1-Aza-3,7-dioxa-2,8-diheptyl-5-methyloxy-bicyclo[3.3.0]octan ca. 13,8 min | |

Nach der Umsetzung gemäß AA3 oder AA4 ist der Peak für das freie 1-Aza-3,7-dioxa-2,8-diheptyl-5-methyloxy-bicyclo[3.3.0]octan bei ca. 13,8 min verschwunden.

### 3. Riechtest

Ein handelsübliches parfümfreies Waschmittel wurde zum einen mit 0,5 Gew.-% des erfindungsgemäßen Kieselsäureesters und zum anderen mit 0,4 Gew.-% des freien Duftstoffs versetzt. Mit jeweils 150g dieser Pulver wurde die Wäsche bei 60 °C gewaschen und 3x mit klarem Wasser nachgespült. Nach dem Schleudern wurde der Duft der feuchten Wäsche beurteilt, danach die Wäsche auf der Leine getrocknet. Der Duft der trockenen Wäsche wurde sofort, nach 1 sowie nach 7 Tagen beurteilt, wobei die Wäsche in Plastikbeuteln gelagert wurde.
Beurteilt wurden die Wäschepaare im direkten Vergleich hinsichtlich der Duftintensität von 7 Fachleuten (Parfümeuren). Die Ergebnisse dieser Tests zeigt Tabelle 1.

### Definition der Skalierung:

- 5: sehr gut zu riechen
- 4: gut zu riechen
- 3: einigermaßen zu riechen
- 2: wenig Duft wahrnehmbar
- 1: kein Duft /kaum Duft wahrnehmbar

**Tablle1: Ergebnisse der Geruchstests weichgespülter Wäsche in Vernel 4-fach**

| | Produkt | Feuchte Wäsche | Tag1 | Tag7 |
|---|---|---|---|---|
| Reiner Duftstoff (Octanal) | 5 | 5 | 3,2 | 2,8 |
| Erfindungsgemäßer Profragrance* | 2,5 | 3 | 4,8 | 3,6 |

| | | | | |
|---|---|---|---|---|
| * 1-Aza-3,7-dioxa-2,8-diheptyl-5-methyloxy-bicyclo[3.3.0]octan-Kieselsäureester | | | | |

## Patentansprüche

1. Kieselsäureester der allgemeinen Formel (I) 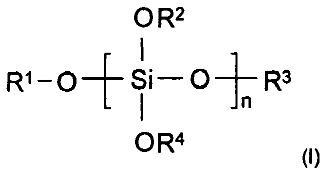 wobei mindestens einer der Reste R¹, R², R³ und R⁴ unabhängig voneinander
(a) eine 1-Aza-3,7-dioxabicyclo[3.3.0]octan-Verbindung der allgemeinen Formel (II), 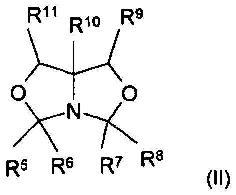
mit R⁵, R⁶, R⁷, R⁸ unabhängig voneinander Wasserstoff, Alkylrest mit 1 bis 8 C-Atomen oder Reste, die in einer Verbindung der allgemeinen Formel R⁵-C(=O)-R⁶ oder R⁷-C(=O)-R⁸ einen Duftstoff, insbesondere einen Duftstoffaldehyd oder ein Duftstoffketon ergeben, wobei R⁵ und R⁶ bzw. R⁷ und R⁸ nicht gleichzeitig Wasserstoff sein können,
R⁹, R¹¹ unabhängig voneinander Wasserstoff, Alkylrest mit 1 bis 8 C-Atomen, Hydroxyalkyl mit 1 bis 8 C-Atomen, Aminoalkyl,
R¹⁰ eine Bindung oder ein divalenter Alkylenrest mit 1 bis 8 C-Atomen,
wobei die Bindung der 1-Aza-3,7-dioxabicyclo[3.3.0]octan-Verbindung der allgemeinen Formel (II) an die Kieselsäureester über den Rest R¹⁰ erfolgt,
und / oder
(b) ein monocyclisches Oxazolidin der allgemeinen Formel (III) darstellt, 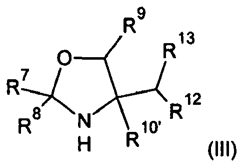
mit den vorstehend angegebenen Bedeutungen für die Reste R⁷, R⁸, R⁹,
R^{10'} eine Bindung oder ein divalenter Alkylenrest mit 1 bis 8 C-Atomen, Wasserstoff, Alkylrest, das durch ein oder zwei Hydroxylgruppen und/oder eine Aminogruppe substituiert sein kann und/oder in dem bis zu 8 nicht benachbarte -CH₂-Gruppen durch -0- ersetzt sein können, und R¹² Wasserstoff, Alkylrest mit 1 bis 8 C-Atomen, Hydroxyalkyl mit 1 bis 8 C-Atomen oder Aminoalkyl,
R¹³ eine OH-Gruppe oder eine Bindung darstellen,
und die noch verbleibenden Reste R¹, R², R³ und R⁴ unabhängig voneinander ausgewählt aus Wasserstoff, Alkylrest mit 1 bis 20 C-Atomen oder Riechstoffalkoholrest,
und n Werte aus dem Bereich von 2 bis 20 annimmt,
wobei die Bindung des monocyclischen Oxazolidins der allgemeinen Formel (III) an die Kieselsäureester über den Rest R^{10'} und oder über den Rest R¹³ erfolgt.

2. Kieselsäureester nach Anspruch 1, **dadurch gekennzeichnet, dass** neben dem Kieselsäureester der allgemeinen Formel (I) eine Mischung aus Kieselsäureester der Formel (VII) vorliegt, 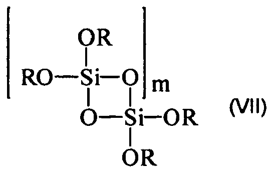 wobei die Reste R unabhängig voneinander die Bedeutungen der Reste R¹, R², R³ oder R⁴ gemäß Formel (I) annehmen und m eine Zahl von 2 bis 20 ist.

3. Kieselsäureester nach Anspruch 2, **dadurch gekennzeichnet, dass** die Mischung Kieselsäureester der Formeln (VIII) und / oder (IX) 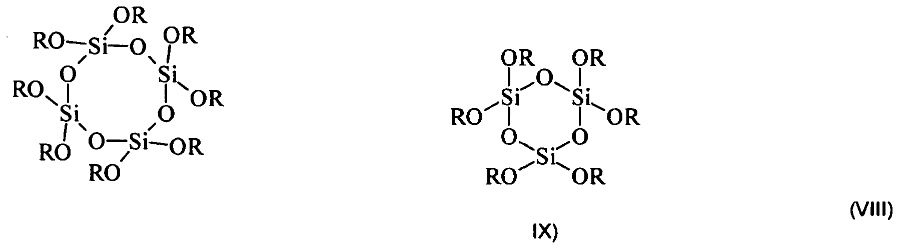 enthält, wobei die Reste R unabhängig voneinander die Bedeutungen der Reste R¹, R², R³ oder R⁴ gemäß Formel (I) annehmen.

4. Kieselsäureester nach einem der vorhergehenden Ansprüche 1 bis 3, **dadurch gekennzeichnet, dass** mindestens zwei der Reste R¹, R², R³, R⁴ eine 1-Aza-3,7-dioxabicyclo[3.3.0]octan-Verbindung der allgemeinen Formel (II) darstellen.

5. Kieselsäureester nach einem der vorhergehenden Ansprüche 1 bis 4, **dadurch gekennzeichnet, dass** der Rest R¹⁰ ein divalenter Alkylenrest, bevorzugt Methylen oder Ethylen ist.

6. Kieselsäureester nach einem der vorhergehenden Ansprüche 1 bis 5, **dadurch gekennzeichnet, dass** mindestens eines der Strukturelemente -CR⁵R⁶ oder -CR⁷R⁸ in einer Verbindung der allgemeinen Formel
R⁵-C(=O)-R⁶ oder R⁷-C(=O)-R⁸ einen Duftstoff ergibt.

7. Kieselsäureester nach einem der vorhergehenden Ansprüche 1 bis 6, **dadurch gekennzeichnet, dass** der Rest R^{10'} ein divalenter Alkylenrest, bevorzugt Methylen oder Ethylen ist.

8. Kieselsäureester nach Anspruch 1, **dadurch gekennzeichnet, dass** der Rest R¹³ eine Bindung darstellt.

9. Wasch- oder Reinigungsmittel enthaltend Kieselsäureester nach einem der vorhergehenden Ansprüche 1 bis 8.

10. Kosmetisches Mittel zur Haar- oder Hautbehandlung, **dadurch gekennzeichnet, dass** es Kieselsäureester nach einem der Ansprüche 1 bis 8 enthält.

11. Verwendung von Kieselsäureester nach einem der Ansprüche 1 bis 8 zur Verlängerung der Duftwirkung von Duftstoffen.

12. Verfahren zur Verlängerung des Duftempfindens von Wasch- oder Reinigungsmitteln, Weichspülern oder Kosmetika oder mit diesen behandelten festen Oberflächen, **dadurch gekennzeichnet, dass** man Kieselsäureester nach einem der Ansprüche 1 bis 8 oder Mischungen daraus zu den Wasch- oder Reinigungsmitteln, Weichspülern oder kosmetischen Mittel zugibt.

## Claims

1. A silicic acid ester of the general formula (I) 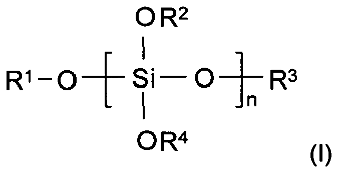 wherein at least one of the residues R¹, R², R³ and R⁴ independently of one another is
(a) a 1-aza-3,7-dioxabicyclo[3,3,0]octane compound of the general formula (II), 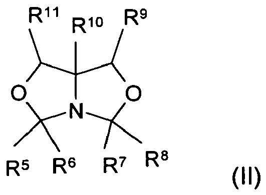
where R⁵, R⁶, R⁷, R⁸ independently of one another are hydrogen, an alkyl residue with 1 to 8 carbon atoms or residues that yield a scent, in particular a scent aldehyde or a scent ketone in a compound of the general formula R⁵-C(=O)-R⁶ or R⁷-C(=O)-R⁸, where R⁵ and R⁶ resp. R⁷ and R⁸ may not be hydrogen at the same time,
R⁹, R¹¹ independently of one another are hydrogen, an alkyl residue with 1 to 8 carbon atoms, hydroxyalkyl with 1 to 8 carbon atoms, aminoalkyl,
R¹⁰ is a bond or a divalent alkylene residue with 1 to 8 carbon atoms,
wherein the 1-aza-3,7-dioxabicyclo[3,3,0]octane compound of the general formula (II) is bonded to the silicic acid ester via residue R¹⁰,
and/or
(b) a monocyclic oxazolidine of general formula (III), 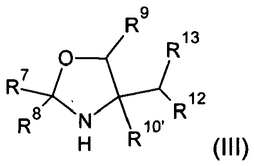
with the meanings given above for the residues R⁷, R⁸, R⁹,
R^{10'} is a bond or a divalent alkylene residue with 1 to 8 carbon atoms, hydrogen, an alkyl residue that may be substituted by one or two hydroxyl groups and/or an amino group and/or in which up to 8 nonvicinal -CH₂ groups may be replaced by -O-,
and R¹² is hydrogen, an alkyl residue with 1 to 8 carbon atoms, hydroxyalkyl with 1 to 8 carbon atoms or aminoalkyl,
R¹³ is an OH group or a bond,
and the remaining residues R¹, R², R³ and R⁴ independently of one another are selected from hydrogen, an alkyl residue with 1 to 20 carbon atoms, or a fragrance alcohol residue,
and n assumes values in the range of 2 to 20,
wherein the monocyclic oxazolidine of the general formula (III) is bonded to the silicic acid ester via the residue R^{10'} and/or via the residue R¹³.

2. The silicic acid ester according to Claim 1, **characterized in that**, in addition to the silicic acid ester of general formula (I), there is a mixture of silicic acid esters of formula (VII), 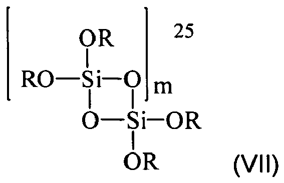 where the residues R independently of one another assume the meanings of the residues R¹, R², R³ or R⁴ according to formula (I) and m is a number from 2 to 20.

3. The silicic acid ester according to Claim 2, **characterized in that** the mixture contains silicic acid esters of formulae (VIII) and/or (IX): 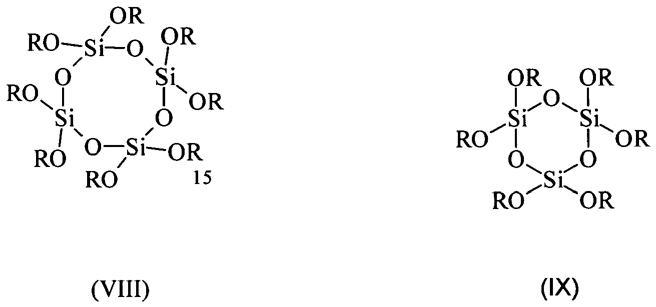 wherein the residues R independently of one another assume the meanings of residues R¹, R², R³ or R⁴ according to formula (I).

4. The silicic acid ester according to any one of the preceding Claims 1 to 3, **characterized in that** at least two of the residues R¹, R², R³, R⁴ are a 1-aza-3,7-dioxa-bicyclo[3,3,0]octane compound of general formula (II).

5. The silicic acid ester according to any one of the preceding Claims 1 to 4, **characterized in that** the residue R¹⁰ is a divalent alkylene residue, preferably methylene or ethylene.

6. The silicic acid ester according to any one of the preceding Claims 1 to 5, **characterized in that** at least one of the structural elements -CR⁵R⁶ or -CR⁷R⁸ in a compound of the general formula
R⁵-C(=O)-R⁶ or R⁷-C(=O)-R⁸ yields a scent.

7. The silicic acid ester according to any one of the preceding Claims 1 to 6, **characterized in that** the residue R^{10'} is a divalent alkylene residue, preferably methylene or ethylene.

8. The silicic acid ester according to Claim 1, **characterized in that** the R¹³ residue is a bond.

9. A washing or cleaning agent containing silicic acid ester according to any one of the preceding Claims 1 to 8.

10. A cosmetic agent for treatment of hair or skin, **characterized in that** it contains silicic acid ester according to any one of Claims 1 to 8.

11. A use of silicic acid ester according to any one of Claims 1 to 8 to prolong the scenting effect of scents.

12. A method for prolonging the scent perception of washing or cleaning agents, fabric rinses or cosmetics or solid surfaces treated with these products, **characterized in that** silicic acid ester according to any one of Claims 1 to 8 or mixtures thereof is/are added to the washing or cleaning agents, fabric softeners or cosmetic agents.

## Revendications

1. Esters d'acide silicique répondant à la formule générale (I) 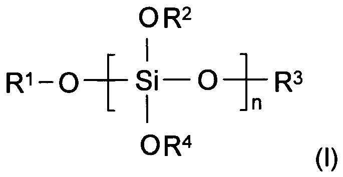 dans laquelle au moins un des résidus R¹, R², R³ et R⁴ représente, de manière respectivement indépendante
(a) un composé de 1-aza-3,7-dioxabicyclo[3.3.0]octane répondant à la formule générale (II) 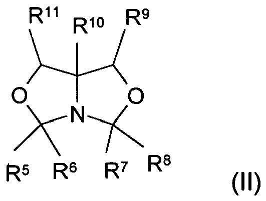
dans laquelle R⁵, R⁶, R⁷, R⁸ représentent, de manière respectivement indépendante, un atome d'hydrogène, un résidu alkyle contenant de 1 à 8 atomes de carbone, ou bien des résidus qui, dans un composé répondant à la formule générale R⁵-C(=O)-R⁶ ou R⁷-C(=O)-R⁸, produisent une substance odoriférante, en particulier un aldéhyde odoriférant ou une cétone odoriférante, R⁵ et R⁶, respectivement R⁷ et R⁸ ne pouvant pas représenter simultanément un atome d'hydrogène ;
R⁹, R¹¹ représentent, de manière respectivement indépendante, un atome d'hydrogène, un résidu alkyle contenant de 1 à 8 atomes de carbone, un groupe hydroxyalkyle contenant de 1 à 8 atomes de carbone, un groupe aminoalkyle ;
R¹⁰ représente une liaison ou un résidu alkylène divalent contenant de 1 à 8 atomes de carbone,
la liaison du composé de 1-aza-3,7-dioxabicyclo[3.3.0]octane répondant à la formule générale (II) aux esters d'acide silicique ayant lieu via le résidu R¹⁰ ;
et/ou représente
(b) une oxazolidine monocyclique répondant à la formule générale (III) 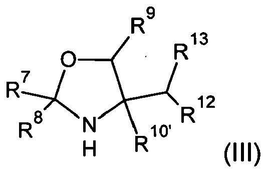
possédant les significations indiquées ci-dessus pour les résidus R⁷, R⁸, R⁹;
R^{10'} représente une liaison ou un résidu alkylène divalent contenant de 1 à 8 atomes de carbone, un atome d'hydrogène, un résidu alkyle qui peut être substitué par un ou deux groupes hydroxyle et/ou par un groupe amino et/ou dans laquelle jusqu'à 8 groupes -CH₂- non voisins peuvent être remplacés par un atome d'oxygène ; et
R¹² représente un atome d'hydrogène, un résidu alkyle contenant de 1 à 8 atomes de carbone, un groupe hydroxyalkyle contenant de 1 à 8 atomes de carbone ou un groupe aminoalkyle ;
R¹³ représente un groupe OH ou une liaison ;
et les résidus R¹, R², R³ et R⁴ qui subsistent encore sont choisis, de manière respectivement indépendante, parmi un atome d'hydrogène, un résidu alkyle contenant de 1 à 20 atomes de carbone ou bien un résidu d'alcool odorant ; et
n prend des valeurs dans la plage de 2 à 20 ;
la liaison de l'oxazolidine monocyclique répondant à la formule générale (III) aux esters d'acide silicique ayant lieu via le résidu R^{10'} ou via le résidu R¹³.

2. Esters d'acide silicique selon la revendication 1, **caractérisé en ce que**, à côté de l'ester d'acide silicique répondant à la formule générale (I), est présent un mélange d'esters d'acide silicique répondant à la formule (VII) 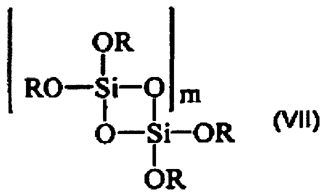 dans laquelle les résidus R prennent, de manière respectivement indépendante, les significations des résidus R¹, R², R³ ou R⁴ selon la formule (I) et m représente un nombre de 2 à 20.

3. Esters d'acide silicique selon la revendication 2, **caractérisé en ce que** le mélange contient des esters d'acide silicique répondant aux formules (VIII) et/ou (IX) 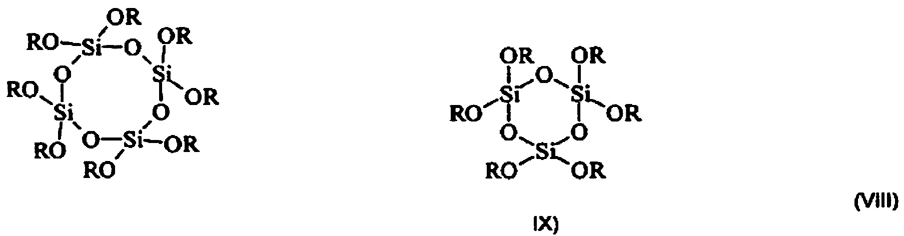 dans lesquelles les résidus R prennent, de manière respectivement indépendante, les significations des résidus R¹, R², R³ ou R⁴ selon la formule (I).

4. Esters d'acide silicique selon l'une quelconque des revendications précédentes 1 à 3, **caractérisés en ce qu'**au moins deux des résidus R¹, R², R³, R⁴ représentent un composé de 1-aza-3,7-dioxabicyclo[3.3.0]-octane répondant à la formule générale (II).

5. Esters d'acide silicique selon l'une quelconque des revendications précédentes 1 à 4, **caractérisés en ce que** le résidu R¹⁰ représente un radical alkylène divalent, de préférence un radical méthylène ou un radical éthylène.

6. Esters d'acide silicique selon l'une quelconque des revendications précédentes 1 à 5, **caractérisés en ce qu'**au moins un des éléments de structure -CR⁵R⁶ ou -CR⁷R⁸ dans un composé répondant à la formule générale R⁵-C(=O)-R⁶ ou R⁷-C(=O)-R⁸ produit une substance odoriférante.

7. Esters d'acide silicique selon l'une quelconque des revendications précédentes 1 à 6, **caractérisés en ce que** le résidu R^{10'} représente un radical alkylène divalent, de préférence un radical méthylène ou un radical éthylène.

8. Esters d'acide silicique selon la revendication 1, **caractérisés en ce que** le résidu R¹³ représente une liaison. ,

9. Agent de lavage ou de nettoyage contenant des esters d'acide silicique selon l'une quelconque des revendications précédentes 1 à 8.

10. Agent cosmétique pour le traitement capillaire ou cutané, **caractérisé en ce qu'**il contient des esters d'acide silicique selon l'une quelconque des revendications 1 à 8.

11. Utilisation d'esters d'acide silicique selon l'une quelconque des revendications 1 à 8 pour prolonger l'effet aromatisant de substances odoriférantes.

12. Procédé pour prolonger la sensation aromatisante d'agents de lavage ou de nettoyage, d'adoucissants ou d'agents cosmétiques ou de surfaces solides traitées avec eux, **caractérisé en ce qu'**on ajoute des esters d'acide silicique selon l'une quelconque des revendications 1 à 8 ou leurs mélanges à des agents de lavage ou de nettoyage, à des adoucisseurs ou à des agents cosmétiques.
